# EUROPEAN PATENT SPECIFICATION

(11) **EP 4 543 526 B1**
(45) Date of publication and mention of the grant of the patent: **11.11.2026**
(21) Application number: 23827844.4
(22) Date of filing: 22.06.2023
(51) Int. Cl.: A61M 25/04, A61M 27/00

(54) **CEREBROSPINAL FLUID SHUNT DEVICES**
VORRICHTUNGEN FÜR LIQUORSHUNT
DISPOSITIFS DE DÉRIVATION DU LIQUIDE CÉPHALO-RACHIDIEN

(30) Priority: 23.06.2022 US 202263354731 P
(43) Date of publication of application: 30.04.2025
(73) Proprietor: MAYO FOUNDATION FOR MEDICAL EDUCATION AND RESEARCH, Rochester, MN 55905 (US)
(72) Inventor: BRINJIKJI, Waleed, Rochester, Minnesota 55902 (US); SAVASTANO, Luis E., Hillsborough, CA, 94010 (US); KALLMES, David F., Rochester, Minnesota 55902 (US); ELDER, Benjamin, Rochester, Minnesota 55902 (US)
(74) Representative: Fish & Richardson P.C.
(86) International application number: PCT/US2023/025984
(87) International publication number: WO 2023/250086

(56) References cited:
- US-A1- 2006 047 336
- US-A1- 2008 249 458
- US-A1- 2008 249 458
- US-A1- 2008 262 406
- US-A1- 2008 262 406
- US-A1- 2019 192 834
- US-A1- 2020 085 600
- US-A1- 2020 085 600

## Description

This application claims the benefit of U.S. Provisional Application Serial No. 63/354,731 filed June 23, 2022.

### TECHNICAL FIELD

The present disclosure relates to catheters, and more particularly, to catheters for accessing cerebrospinal fluid.

### BACKGROUND

Cerebrospinal fluid (CSF) flows through ventricles and bathes the brain and spinal column. Hydrocephalus, idiopathic intracranial hypertension, and other CSF dynamics disorders relate to a buildup of CSF from increased production or decreased absorption, with deleterious effects on the brain. To treat these disorders, CSF may be diverted away from the brain to normalize the CSF pressure around the brain. However, many CSF diversion technologies, for example shunts, require invasive spine or brain surgery and have high revisions rates. In one example, a procedure of implanting a shunt through a vein in the skull and intro the subarachnoid space at a cerebellopontine angle cistern introduces a great risk of subarachnoid hemorrhage and injury to the brain and brainstem. Such procedure is limited as there are only two possible implantation sites and there are critical structured at high risk of injury such a brainstem, cranial nerves and arteries and veins, which can be potentially catastrophic consequences if injured during shunt implantation. Accordingly, it may be desirable to provide an endovascular procedure to access the intradural space at the spine to facilitate evacuation and drainage of CSF, delivery of drugs or therapeutic agent, and delivery or implantation of a device.

US 2008/262406 A1 describes an implantable system for securing shunt catheters to maintain shunt patency, and thus, prevent shunt malfunction due to obstruction.

US 2020/085600 A1 describes methods and devices for creating fluid connections between bodily chambers or vessels that are not naturally connected.

US 2008/249458 A1 describes a device for use in opening obstructions within the brain.

### SUMMARY

The present invention is defined by the independent claim 1. The dependent claims depict additional embodiments of the invention.

The present disclosure describes systems and devices for accessing the intradural spinal space and shunting CSF to the venous system. The intradural spinal space includes the occipito-cervical level, the cervical level, the thoracic level, the lumbar level, and the sacro-coccigeal level. The present disclosure describes devices for diverting CSF intravenously in a way that mimics what happens physiologically. In particular, in some embodiments the devices described herein create a fluid passageway (or act as a shunt device) in a lower spinal region to drain CSF into a venous system of a patient.

The devices and systems described herein are a platform that provides access to the intradural space from the intra-vascular venous compartment, prevents blood extravasation while the passageway is patent (open), enables navigation within the intrathecal spinal compartment without spinal cord or nerve rootlets damage, and allows temporary or permanent placement of a catheter for CSF extracorporeal drainage and or a shunt for intravascular diversion.

In a first example aspect, a shunt catheter device for draining cerebrospinal fluid (CSF) into a venous system of a lower spinal region includes a sheath (14) having an interior volume (18), a catheter (22) disposed in the interior volume (18) of the sheath (14) and movable relative to the sheath (14), a first flange (26) coupled to the catheter (22) and disposed between the catheter (22) and the sheath (14), and a second flange (30) proximally located relative to the first flange (26) and disposed between the catheter (22) and the sheath (14). The first (26) and second (30) flanges are expandable between a compressed configuration when the first (26) and second (30) flanges are disposed in the interior volume (18) of the sheath (14), and an expanded configuration when the first (26) and second (30) flanges are externally disposed relative to the sheath (14). The first flange (26) is separably deliverable relative to the second flange (30).

The shunt catheter device may include any one or more of the following forms.

In one example, the first (26) and second (30) flanges may be a Nitinol braided material.

In another example, the first (26) and second (30) flanges may be attached to the catheter.

In some examples, a check valve (56) may be operably coupled to the catheter (22) and disposed downstream from the first (26) and second (30) flanges.

In other examples, the catheter (22) may have a length in a range of 10 cm to 20 cm.

In yet another example, the catheter (22) may have an inner diameter in a range of 0.5 mm to 2.0 mm.

In some examples, one or more of the first (26) and second (30) flanges may have an outer diameter in a range of 2.5 mm to 5 mm in the expanded configuration.

In other examples, one or more of the first (26) and second (30) flanges may be coupled to the catheter (22) by spot-welding.

In one example, one or more of the first (26) and second (30) flanges may be coupled to the catheter (22) by stitching.

Other features and advantages of the present disclosure will be apparent from the following detailed description, the figures, and the claims.

### BRIEF DESCRIPTION OF THE DRAWINGS

The following drawings illustrate certain example embodiments, features and advantages of the devices, systems, and non-claimed methods described in this disclosure. These illustrations are not intended to limit the scope of the inventive concepts described herein. Like reference symbols in the drawings indicate like elements.
Fig. 1 is a side view of a first example catheter device assembled in accordance with the teachings of the present disclosure, the catheter device in a pre-delivery configuration;
Fig. 2 is a side view of the catheter device of Fig. 1, the catheter device in a delivered configuration;
Fig. 3 illustrates the catheter device of Fig. 1 implanted in a lower spinal region of a patient;
Fig. 4 is a side view of a second example catheter device assembled in accordance with the teachings of the present disclosure;
Fig. 5 is cross-sectional view of a CSF implant coupled to a venous implant, the implants delivered to a CSF space and vein of a patient using the catheter device of Fig. 4;
Fig. 6 is a side view of the implant arrangement of Fig. 5, showing the CSF implant magnetically coupled to the venous implant;
Fig. 7 illustrates the implant arrangement of Fig. 5 implanted in proximity to a cervical spine of a patient;
Fig. 8 is a side view of a third example catheter device assembled in accordance with the teachings of the present disclosure, the catheter device in a compressed configuration;
Fig. 9 is a side view of the catheter device of Fig. 8, the catheter device in an expanded configuration;
Fig. 10 illustrates a location for creating a fluid passageway using the catheter device of Fig. 8 in a lower spinal region of a patient;
Fig. 11 is a schematic diagram of a non-claimed method of creating a fluid passageway in a lower spinal region to drain CSF fluid into a venous system of a patient;
Fig. 12 is a side view of a fourth example shunt device assembled in accordance with the teachings of the present disclosure;
Fig. 13 illustrates a catheter creating a fluid passageway before delivering the shunt device of Fig. 12 in a lower spinal region of a patient;
Fig. 14 illustrates the shunt catheter device of Figs. 11 and 12, the shunt catheter device in a delivery configuration;
Fig. 15 illustrates the shunt catheter device of Fig. 12 implanted in a lower spinal region of a patient;
Figs. 16A through 16H illustrate the anatomic approach to accessing the neural foraminal vein with a guide catheter followed by the shunt delivery catheter and dural perforating element;
Figs. 17A through 17H illustrate another approach to delivering the shunt catheter;
Figs. 18A through 18D illustrate another approach to delivering the shunt catheter whereby a wire is first advanced to the epidural plexus;
Figs. 19A through 19D illustrate an over-the-wire delivery of the delivery catheter to the epidural vein;
Figs. 20A and 20B illustrate the anatomical configuration of the foraminal veins, anterior and posterior epidural plexus, and their relation to the lumbar CSF space and fluoroscopically visible bone structures including the vertebral body, pedicles and lamina;
FIG. 21 illustrates an example Bovie knife (or Bovie wire) that can be used to perforate, cut, or destroy tissue (e.g., dura) to create a passageway for the delivery and implantation of the catheter shunt devices described herein;
Fig. 22 illustrates an example delivery/push catheter that can be used to advance and deploy a detachable shunt catheter device as described herein;
Fig. 23 illustrate the implant suture point of the delivery/push catheter of Fig. 22 and its attachment to an example shunt catheter device;
Fig. 24 shows an example shunt catheter device construction in accordance with some embodiments;
Fig. 25 shows another example design of a shunt catheter device in accordance with some embodiments;
Fig. 26 shows another example design of a shunt catheter device in accordance with some embodiments;
Fig. 27 schematically shows a shunt catheter device with a portion anchored in the thecal sac and communicating with the venous system;
Fig. 28 shows an example system that includes shunt catheter device as a self-expanding EPTFE covered nitinol hypotube in accordance with some embodiments; and
Fig. 29 shows the shunt catheter of Fig. 28 in a radially expanded configuration.

### DETAILED DESCRIPTION

The present disclosure describes systems, devices, and non-claimed methods for use in minimally invasive surgical procedures enabling transvascular neurosurgery without opening the spine. For example, the systems, devices, and non-claimed methods described herein improve access to the intradural space of the spine to facilitate drainage or diversion of CSF to the venous system, delivery of drugs or therapeutic agents, and the delivery or implantation of a device. Each level and each venous component of the veins of the spine require novel device mechanisms both for accessing the spinal venous system, perforating into the CSF space, as well as controlling the drainage of CSF.

Those skilled in the art will recognize that the non-claimed methods and devices described herein also apply to percutaneous, minimally invasive, laparoscopic, endoscopic, and/or open surgical access to the spinal venous system.

In addition, the non-claimed methods described herein for a particular level would also apply to any other spinal level bilaterally. In addition, those skilled in the art will recognize that the non-claimed methods and devices described herein also apply to trans-arterial access to the spine employing segmental arteries, spinal branches and other branches.

The preferred embodiments described herein are not meant to limit other embodiments, non-claimed methods or devices at any level of the spine including the occipito-cervical region.

### Anatomy of Spinal Veins

There are several anatomical pathways to accessing the spinal veins, including extradural and para-spinal compartments, as described further below.

***Extradural Compartment.*** The extradural compartment refers to the internal vertebral venous plexus, also known as the epidural plexus. It sits within the spinal canal, embedded in the epidural fat that surrounds the main thecal sac, and drains the spinal cord and the vertebral bodies. Although the internal venous plexus effectively surrounds the dura circumferentially, *Gray's Anatomy of the Human Body* describes four longitudinal channels where the plexus is more prominent, two anteriorly and two posteriorly. Radiologically, superimposition of the anterior and posterior plexuses on a frontal view results in the appearance of two "lateral epidural plexuses" running longitudinally on either side of the spinal canal. The diameter of the epidural venous plexus ranges from 1-3mm.

Venous rings at the midlevel of each vertebral body allow communication between the two anterior and two posterior internal plexuses. On a frontal view, these appear as transverse channels connecting the left and right lateral plexuses. These rings also drain the basivertebral veins from the posterior aspect of the vertebral bodies into the epidural plexus. These osteal tributaries are morphologically distinct on fluoroscopy. Awareness of their existence and relationship to the epidural plexus can help distinguish them from epidural veins during navigation and positioning of endovascularly placed shunt catheters. The epidural plexus is both rich and valve-less. The mean pressure in the epidural venous plexus is from 12 mmHg (1600 Pa). For the conversion 1 mmHg is equivalent to 133.322 pascals (Pa).

***Paraspinal Compartment.*** The paravertebral compartment is also referred to as the external vertebral, paravertebral, or paraspinal venous plexus. It is richest in the cervical region and is also divided into two anterior and two posterior columns that freely communicate with each other. The anterior external plexuses lie adjacent to the vertebral bodies and also drain the aforementioned basivertebral and foraminal veins. The posterior external plexuses run outside the lamina and drain the deep para-spinal muscles via tributaries such as the lateral and posterior muscular veins. They also communicate with the posterior internal plexuses and, in the cervical region, anastomose with the vertebral, occipital, and deep cervical veins.

The internal and external vertebral plexuses are functionally and anatomically connected through each intervertebral foramen via the foraminal veins which form a plexus surrounding each nerve root. These are also referred to by the term "intervertebral vein." Both micro-catheters and embolic agents injected here behave more like a plexus.

In the thoracolumbar spine, these veins drain directly into the adjacent segmental vein (such as an intercostal vein) at each level, and are useful for navigating a microcatheter or wire as close as possible to the nerve root sleeve or epidural plexus for delivery of the shunt catheter. The mean pressure in the para-spinal venous plexus is from 11 mmHg (1467 Pa).

***Efferent.*** The term "segmental vein" is a general term encompassing the posterior intercostal, subcostal, and lumbar veins. "Para-spinal vein" refers to the part of the segmental vein that runs adjacent to the vertebral body. It effectively begins medial to where the foraminal vein joins the intercostal or lumbar vein and ends by draining into a larger vein such as the azygos vein.

This segmental arrangement, in practice, is complex, with intersegmental veins anastomosing adjacent segments. The onward drainage of each segmental vein varies in different parts of the spine. The next section covers the gross organization of efferent pathways from segmental veins at various levels toward the superior vena cava (SVC). The mean pressure in the segmental veins is around 9-10 mmHg (1200-1333 Pa).

***Azygous Vein.*** The azygos system comprises the azygos vein on the right and the hemiazygos (lower thoracic) and accessory hemiazygos (upper thoracic) veins on the left. It connects the SVC and IVC and serves as an alternative route for venous return to the heart when either the SVC or IVC is compromised.

The azygos vein is a right-sided longitudinal paravertebral structure in the thorax. It is formed by the union of the right ascending lumbar vein with the right subcostal vein at the level of T12. From there, it ascends in the posterior mediastinum before coursing anteriorly to form an arch above the right main bronchus and draining into the posterior aspect of the SVC. Conventionally, there is a single channel, though primitive duplicate channels can persist. Although classically described as a right-sided structure, the azygos vein can run in the midline or even slightly to the left. Apart from draining the right side of the posterior trunk, the azygos vein also carries the burden of draining blood from its left-sided counterparts into the SVC. On the left, the hemiazygos vein drains the ascending lumbar vein, left subcostal, and 9th-to-11th intercostal veins, usually coursing around the anterior aspect of the T9 vertebral body to drain into the azygos vein. The accessory hemiazygos vein usually drains the fifth-to-eighth intercostal veins on the left, before also crossing the midline at the level of T8 to drain into the azygos vein. The hemiazygos and accessory hemiazygos veins may also be directly connected to each other. The pressure in the azygouns vein ranges from 8-10 mmHg (1067-1333 Pa).

***Vertebral Veins.*** Unlike in the thorax and abdomen, the skeletal arteries and veins in the neck are not arranged in a strictly metameric fashion. A rich venous network surrounds the cervical spine and communicates with the internal jugular vein and the venous plexuses surrounding the foramen magnum and skull base. The vertebral veins are paired longitudinal paravertebral structures that drain the cervical intervertebral foramina and paravertebral plexuses. In the lower neck, each vertebral vein usually exists as a uniform single channel, but higher up, its configuration consists of a rich, confluent, and valve-less paravertebral plexus. This plexus begins at the level of C1, surrounds the vertebral artery within the transverse foramina of C2-C6, and funnels inferiorly into a large, single channel at the level of C6. Medially, the paravertebral plexus is continuous with the epidural plexus within the spinal canal via the foraminal veins. Vertebral venous system has a pressure similar to the internal jugular vein (6-10 mmHg, 800-1333 Pa). The vertebral vein can be used as a means to access the suboccipital venous plexus and the foramen magnum and marginal sinus.

***Ascending Lumbar Vein.*** In the lumbar spine, the segmental veins converge to form the ascending lumbar veins on either side of the midline. While the right ascending lumbar vein ends in the origin of the azygos vein, the left ascending lumbar vein can either cross the midline to drain directly into the azygos vein or remain to the left of the midline to join the left subcostal vein in forming the hemiazygos vein. The left ascending lumbar vein can be accessed directly from the left common iliac vein. The pressure in the common iliac vein is from 12-18 mmHg (1600-2400 Pa), an ideal pressure for equilibration with the CSF space.

The present disclosure provides an approach to deploying the shunt catheter devices described herein using the epidural venous plexus. There are multiple paths to navigation and delivery of shunt catheters into the epidural venous plexus as described above. Epidural venous plexuses is located in the epidural space. It is an avalvular venous system which allows navigation with endovascular devices for one or more levels. It has numerous anastomoses with the superior and inferior caval venous system providing multiple corridors of access and ensuring outflow of draining CSF in case of focal venous occlusion. This would also be beneficial when more than one access points to the intradural spinal space are needed (for example when more than one CSF shunts has to be implanted, or when a shunt fails and another is required, or when the trans-vascular access to the CSF space at a certain level was unsuccessful and needs to be re-attempted in a different site)

The epidural venous plexuses are formed by the anterior and posterior longitudinal plexuses. The anterior longitudinal plexuses provide a larger pathway to the intradural space. Therefore, one or more elements of the system may include articulating elements or be made with elastic materials pre-shaped to direct the penetrating elements posteriorly towards the dura.

The anterior longitudinal plexuses comprise on each side two longitudinal venous networks, one lateral and the other medial. The anterior epidural venous plexuses are located within connective adipose areolar tissue between the posterior longitudinal ligament and the posterior side of the vertebral body and the dura. The adipose areolar tissue is advantageous for extravascular advancement in some embodiments as it provides a soft tissue, and also separates physically the intravascular and intradural spaces, thereby decreasing the likelihood of iatrogenic CSF/venous fistulization upon removal of the perforation elements.

The two right and left longitudinal plexuses are medialized at the pedicle and lateralized at the intervertebral disc. A medialize perforation point may be beneficial in some embodiments as it decreases the likelihood of encountering nerve roots underlying the dura at the perforation point.

The internal vertebral plexuses are linked by transverse plexuses passing in front of and behind the dural sheath, forming an epidural venous ring that is more developed in its anterior segment. The epidural venous plexuses communicate through the foraminal veins and, depending on the segment considered, with the vertebral, intercostal, lumbar or sacral veins.

According to teachings of the present disclosure, a device, system, and non-claimed methods provide minimally invasive access to the intradural space from the intra-vascular venous compartment, and temporary or permanent placement of a catheter for CSF extracorporeal drainage and or a shunt for intravascular diversion. The device can include: A) a delivery catheter which is typically an elongated tubular element that is navigated from a peripheral venous access site (*e.g.*, femoral vein or jugular vein) into the vertebral venous system; B) a perforating element, which may be a wire or stylet (*i.e.,* without a lumen) or shaft (*i.e.,* an elongated element with a lumen) with cutting or energizable elements to access the spinal CSF space from the venous intravascular access; and C) a shunt or CSF draining tube as an elongated element with a lumen that is delivered over a wire or stylet and/or through a shaft and fluidly couples the spinal CSF space to the intravascular venous space and/or the extracorporeal space (*i.e.,* outside the body).

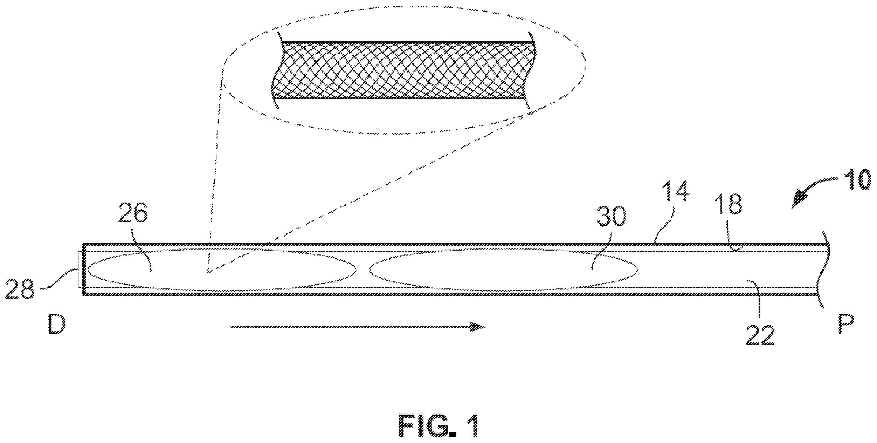

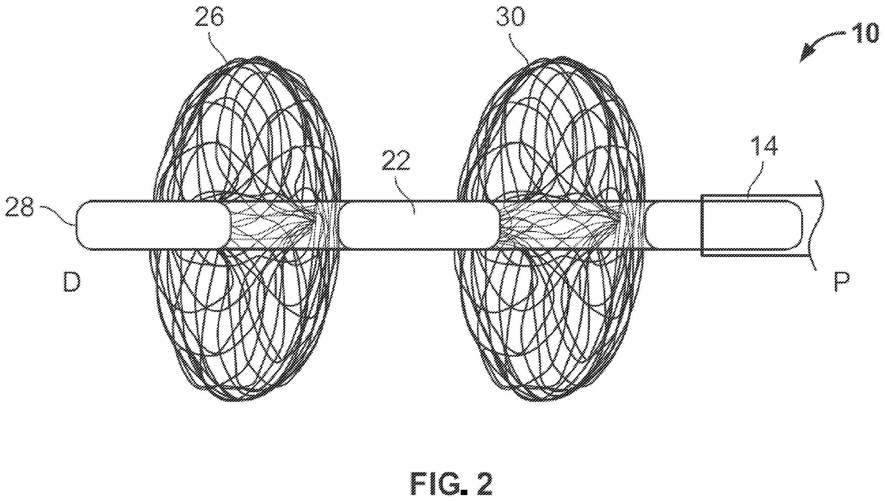

An example shunt catheter device 10 for draining cerebrospinal fluid (CSF) into a venous system of a lower spinal region is shown in Figs. 1-3. As shown in Figs. 1 and 2, the shunt catheter device 10 includes a sheath 14 having an interior volume 18, a catheter 22 disposed in the interior volume 18 of the sheath 14 and being movable relative to the sheath 14. A first flange 26 is coupled to a distal end 28 of the catheter 22 and is disposed between the catheter 22 and the sheath 14. A second flange 30 is proximally located relative to the first flange 26 and is disposed between the catheter 22 and the sheath 14. The first and second flanges 26, 30 are expandable between a compressed configuration, as shown in Fig. 1 when the device 10 is in a pre-delivery configuration and an expanded configuration, as shown in Fig. 2. In the compressed configuration, the first and second flanges 26, 30 are disposed in the interior volume 18 of the sheath 14, providing a low profile for the catheter device 10 to navigate the venous system for delivery. Once the catheter device 10 reaches a target implantation site, the sheath 14 is pulled in a proximal direction P uncovering the first and second flanges 26, 30, and delivering the first and second flanges 26, 30 to the delivery site. In the delivered configuration, the first and second flanges 26, 30 expand and are externally disposed relative to the sheath 14. The first flange 26 is separately deliverable relative to the second flange 30. As will be described below, the catheter 22 advances to a first delivery site in the CSF space where the first flange 26 is delivered (or uncovered by the sheath), and then advances to a second delivery site in a vein where the sheath 14 uncovers the second flange 30 to deliver the second flange 30 in the vein.

The first and second flanges 26, 30 are attached to the catheter 22 so that when the sheath 14 is removed (*i.e.,* pulled in a proximal direction P), the flanges 26, 30 radially expand yet remain attached to the catheter 22. In the illustrated example, the flanges 26, 30 are coaxially aligned with a longitudinal axis of the catheter 22 so that the flanges 26, 30 expand radially from the circumference of the catheter 22. The first and second flanges 26, 30 are coupled to the catheter 22 by spot-welding, stitching, or other suitable non-claimed methods. The catheter 22 has a length in a range of approximately 10 cm to approximately 20 cm, and an inner diameter in a range of approximately 0.5 mm to approximately 2.0 mm. In the expanded configuration, each of the first and second flanges 26, 30 has an outer diameter in a range of approximately 2.5 mm to approximately 5 mm.

The first and second flanges 26, 30 are made of a woven material suitable for implantation. For example, the flanges 26, 30 are made from a Nitinol braided material that has a densely woven weave when in a compressed configuration, as shown in Fig. 1, and that can expand when in a larger environment and hold an expanded configuration, as shown in Fig. 2. Nitinol braided flanges 26, 30 are pliable to compress for assembling the catheter device 10, yet durable to retain the expanded position within a body cavity.

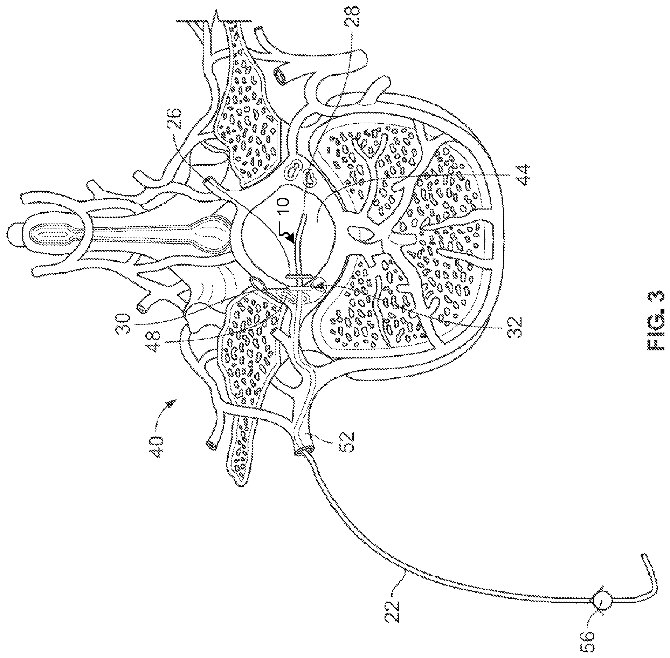

In Fig. 3, a shunt formed by first and second flanges 26, 30 and the catheter 22 of the shunt catheter device 10 of Figs. 1 and 2 are implanted in a lower spinal region 40, such as the lower lumbar spine, of a patient. The flanges 26, 30 are disposed in different areas of the lumbar region, and specifically in a CSF space 44 and a vein 48 in close proximity to the CSF space 44. Once expanded, the flanges 26, 30 create a shunt to form a passageway to permit CSF drainage through the catheter 22 and into the venous system. In particular, the catheter 22 provides a drainage passageway from the CSF space 44 and through the venous system via a common iliac vein 52. After the shunt catheter device 10 is delivered, the catheter 22 is left in the common iliac vein 52 to allow for continued CSF drainage. A check valve 56 is operably coupled to the catheter 22 and disposed downstream from the first and second flanges 26, 30. The check valve 56 permits CSF flow in one direction from the CSF space 44 and into the venous system.

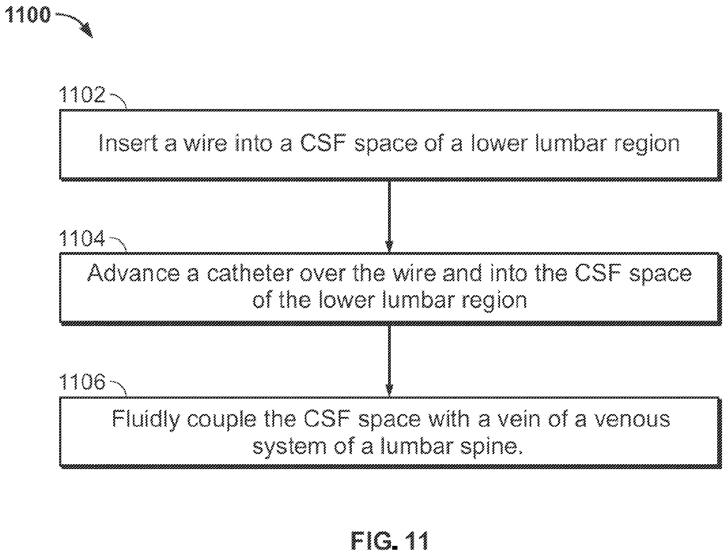

An example method 1100 that is not forming part of the invention is illustrated in Fig. 11. The non-claimed method 1100 includes delivering the shunt catheter device 10 to lower spinal CSF space will be described with respect to Fig. 3. The non-claimed method 1100 applies to other levels of the spine, as well. To deliver the endovascular shunt catheter device 10 and create a fluid passageway in the lower spinal region 40 using the catheter device 10 of Figs. 1 and 2, an operator first inserts 1102 a wire into the CSF space 44 of a lower spinal region 40. The catheter 22 is advanced over the wire and into the vein 52 of the venous system. The operator then advances 1104 the catheter 22 over the wire and into the CSF space 44 of the lower spinal region 40. To fluidly couple the CSF space 44 with the vein 52 of the venous system, the device 10 punctures the vein 52 and a dura disposed between the vein 52 and the CSF space 44 before advancing the catheter into the vein 52 and into the CSF space 44. Once the distal end 28 of the catheter 22 is in the CSF space 44, an operator then delivers a CSF implant, or first flange 26 of the shunt catheter 32, into the CSF space, by sliding the sheath 14 away from the distal end 28 of the catheter 22 and uncovering the first flange 26. The operator then continues to advance the catheter 22 into proper positioning in the vein 52 to deliver a venous implant, or second flange 30 of the shunt catheter 32, by sliding the sheath 14 in a proximal direction P and uncovering the second flange 30. As shown in Fig. 3, the first and second flanges 26, 30 are disposed in the CSF space 44 and vein 52, respectively, and remain coupled to the catheter 22 to form the shunt catheter 32.

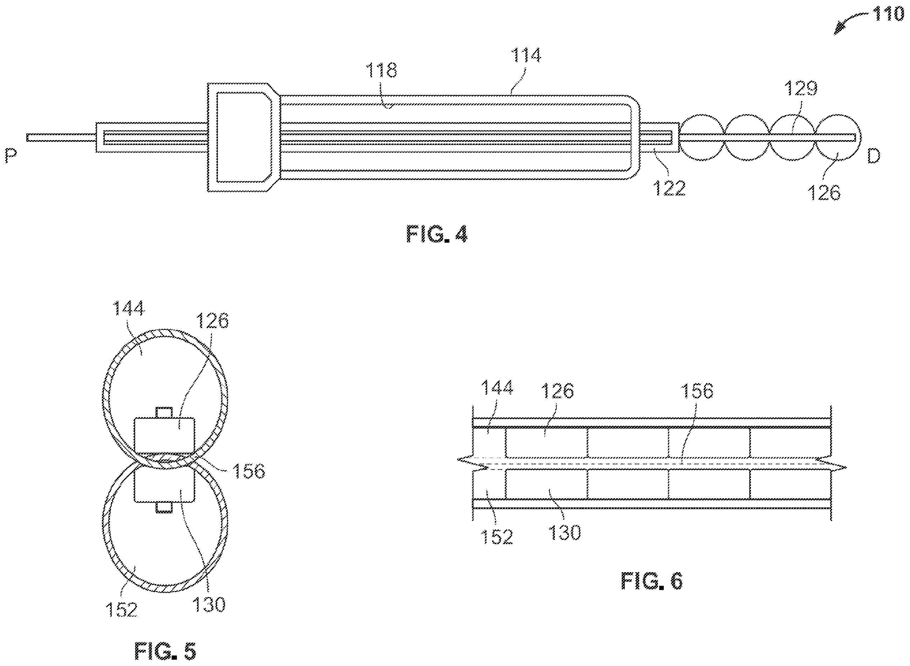

In Fig. 4, a second example shunt catheter device 110 includes a sheath 114 having an interior volume 118, a catheter 122 disposed in the interior volume 118 of the sheath 114 and movable relative to the sheath 114, an implant suture 129, and two or more implantable magnets 126 carried by the implant suture 129. To perform the non-claimed method 1100 of Fig. 11 to implant the shunt catheter device 110, two catheter devices are used: a venous shunt catheter device 110 and a CSF catheter device 110. The catheter devices 110 are substantially identical, and therefore may be represented by the shunt catheter device 110 of Fig. 4. In other words, the CSF catheter device 110 is used separately to deliver a CSF implant 126 into a CSF space 144 from the venous shunt catheter device 110 to deliver venous implant 130 into the vein 152, as shown in Figs. 5-7.

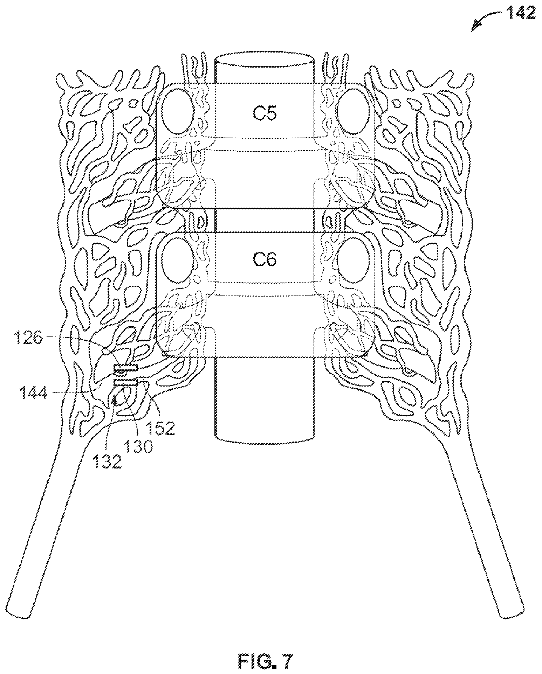

The non-claimed method 1100 in Fig. 11 of using the shunt catheter device 110 will be described with respect to Figs. 5-7. To deliver the endovascular shunt catheter device 110 to the target site and create a fluid passageway in the lower spinal region using the shunt catheter device 110 of Fig. 4, an operator first inserts 1102 a wire into the CSF 144 space of a lower spinal region 142. In the example shown in Fig. 7, the lower spinal region is a cervical spine. The operator then advances 1104 the catheter 122 over the wire and into the CSF space 144 by puncturing a dura of the lower spinal region. Once in the CSF space, an operator then delivers a CSF implant 126 into the CSF space 144 by decoupling one or more CSF implants 126 *(i.e.,* magnets) from the implant suture 129. Delivering the CSF implant includes depositing a magnet into the CSF space. The operator advances a different catheter 122 into the vein 152 and over the wire by puncturing the vein 152 to deliver a venous implant 130 in the vein 152 and in proximity to the delivered CSF implant 126. As shown in Figs. 5 and 6, the implants 126, 130 of the implant arrangement) magnetically couple, pinching the dura 156 and the wall of the vein between the implants 126, 130 to form a fistula. Once the fistula forms, the implants 126, 130 drop away from the implant site, leaving a passageway between the vein 152 and the CSF space 144.

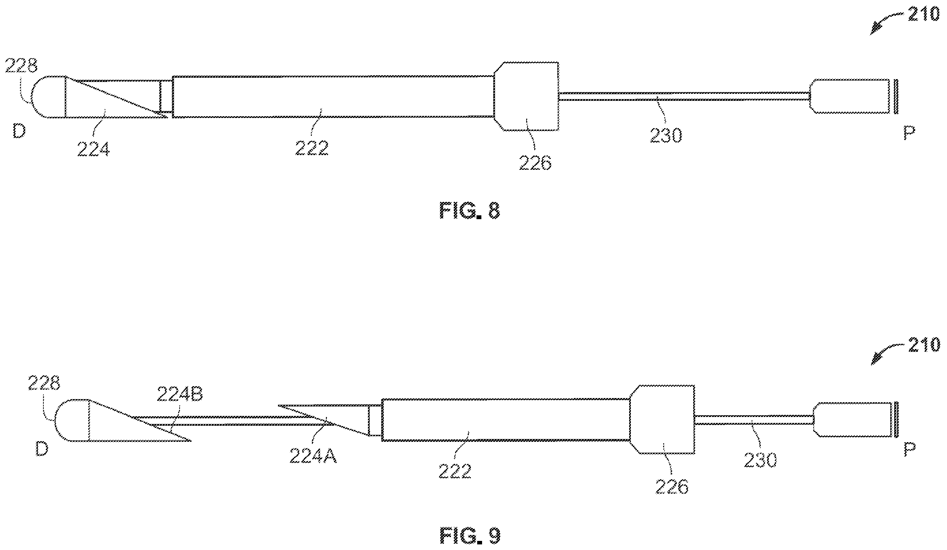

In Figs. 8 and 9, a third example shunt catheter device 210 includes a distal hole punch tip 224, a catheter 222, a catheter hub 226, and a wire 230. The hole punch tip 224 defines the distal end 128 of the catheter device 210 and includes a first blade 224A and a second blade 224B. The first and second blades 224A, 224B are separable to puncture a vein or a dura in forming a fluid passageway. Specifically, once the catheter device 210 is inserted through the venous system of the lower spinal region of the patient, a user may operate the device by separating the first and second blades 224A, 224B sliding the catheter hub 226 in a proximal direction and/or sliding the wire 230 in a distal direction. When the blades 224A, 224B are separated, and a dura is disposed between the blades, the operator may slide the catheter hub 226 in the distal direction and/or slide the wire in the proximal direction to join the blades 224A, 224B and puncture the dura.

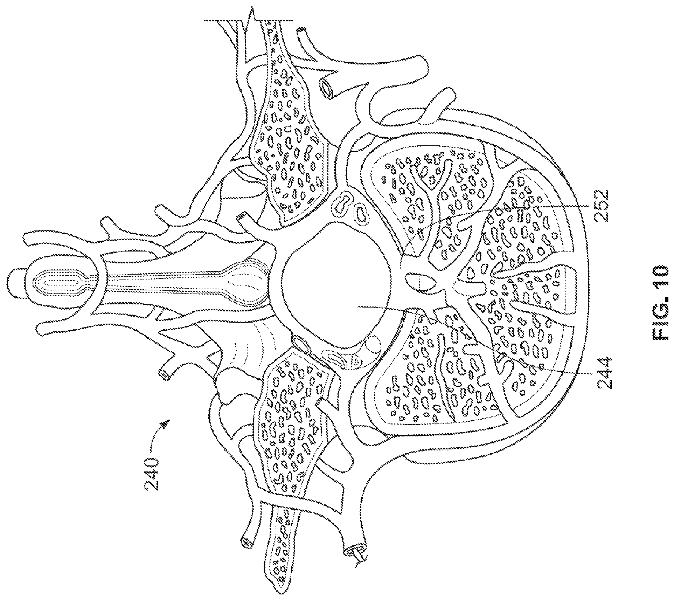

The non-claimed method 1100 in Fig. 11 of percutaneously delivering the shunt catheter device 210 will be described with respect to Fig. 10. To deliver the shunt catheter device 210 and create a fluid passageway in the lower spinal region using the catheter device 210 of Figs. 8 and 9, health care provider may first puncture through a layer of skin of a patient and into the CSF space 244 and into the vein 252 of the lower spinal spine 240. The provider may then insert 1102 a wire into the CSF space 244 of the lower spinal region 240 and advance 1104 the catheter 222 over the wire and into the CSF space 244. To fluidly couple the CSF space 244 with a vein 252 of a venous system of the spine (*i.e.,* create a fluid passageway), the provider may puncture the vein 252 and the dura disposed between the vein 252 and the CSF space 244 before advancing the catheter 222 into the vein 252 and into the CSF space 244. Creating this passageway may include separating the first blade 224A from the second blade 224B attached to the catheter 222 of a catheter device 210 and moving the blades 224A, 224B relative to a distal end 228 of the catheter 222. Once the dura is disposed between the separated blades 224A, 224B, the provider may then puncture the dura between the CSF space 244 and the vein 252 by drawing the blades 224A, 224B together. After the catheter device 210 is removed from the patient, a drainage passageway is formed between the CSF space 244 and the vein 252.

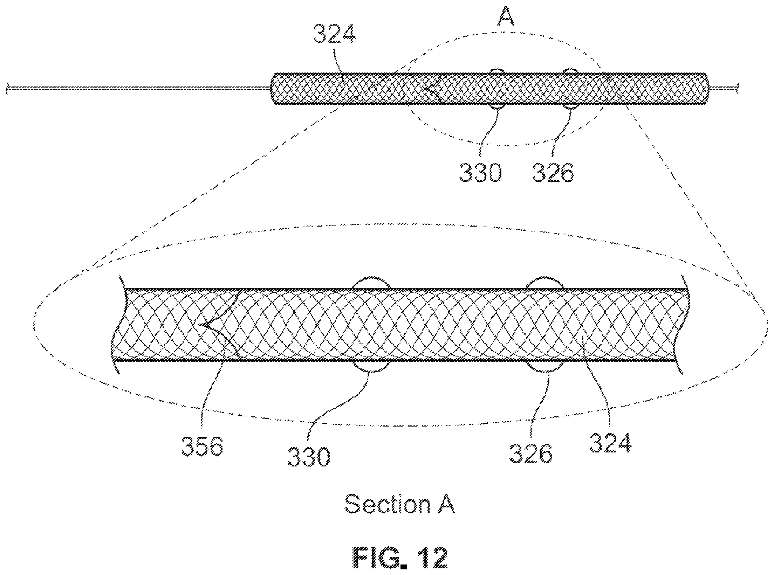

Figs. 12-15 illustrate a third example shunt catheter device 310 constructed in accordance with the teachings of the present disclosure. The third example shunt catheter device 310 is similar to the first example shunt catheter device 10 of Figs. 1-3, with similar reference numerals used for similar components. However, the third example shunt catheter device 310 includes a shunt 324 that may be removably coupled from the catheter 322. The third example shunt catheter device 310 may be used according to the non-claimed method or process 1100 of Fig. 11.

The shunt 324 in Fig. 12 serves as an implantable drain of self-expanding metal with shape memory (*e.g*., Nitinol, thin film Nitinol, cobalt chromium, etc.). The weave of the metal material include a plurality of overlapping struts that are coated so CSF travels through a hollow interior of the shunt 324 and not through or in between the woven struts. The shunt 324 include a first plate 326, a second plate 330, and a check valve 356 built into the shunt 324. When the shunt 324 is implanted, the shunt 324 expands, thereby placing the first plate in a CSF space and the second plate 330 in a venous space.

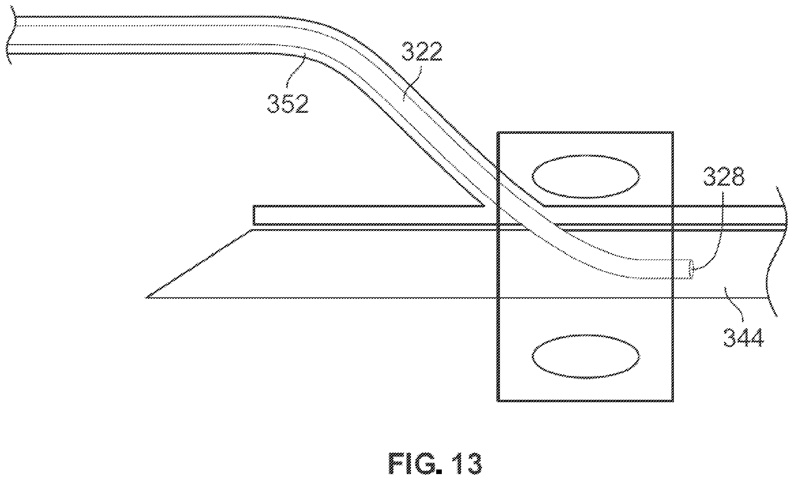

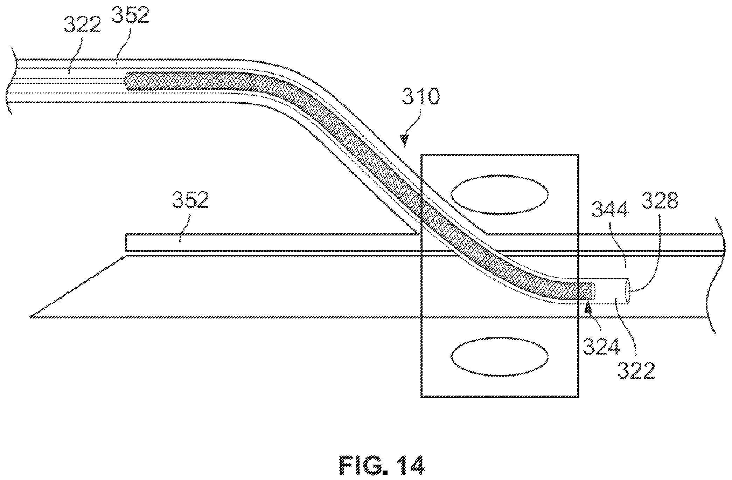

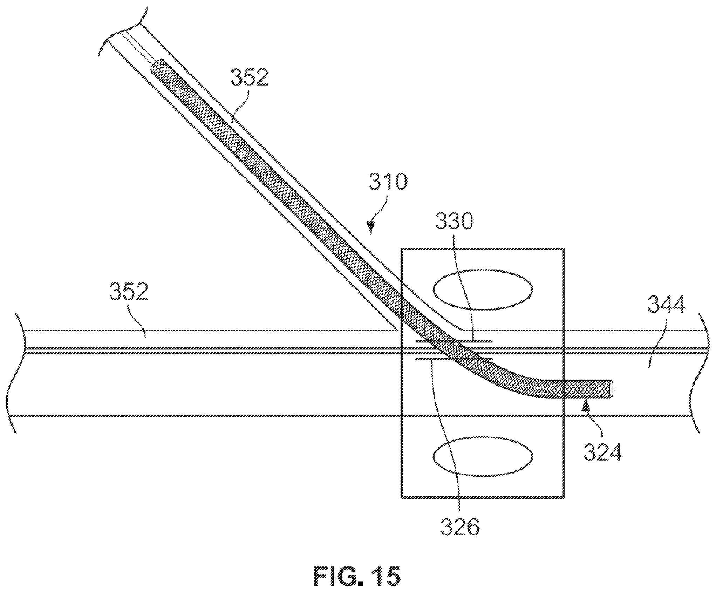

Fig. 13 illustrates a schematic of accessing a CSF space 344 through a vein 352 using a catheter 322 of the shunt catheter device 310. In Fig. 13, a catheter 322 (2-5Fr) is introduced into the CSF space 344 over a guide wire. Once the catheter 322 is in position such that a distal end 328 is disposed in the CSF space 344, the shunt 324 may be inserted into an interior cavity of the catheter 322 and guided to the delivery site, as shown in Fig. 14. To deliver the shunt 324, the catheter 322 may be pulled in a proximal direction and away from the shunt 324 to allow the shunt 324 to expand into the CSF space 344 and the venous space 352. Particularly, in Fig. 15 the shunt 324 is unsheathed by removing the catheter 322 from the shunt 324, disposing first and second plates 326, 330 of the shunt body 324 between the CSF space 344 and the vein 352. The rigidity of the shunt 324 is sufficient for the plates 326, 330 to maintain their position and permit fluid to drain through the hollow body of the shunt 324 and into the venous system.

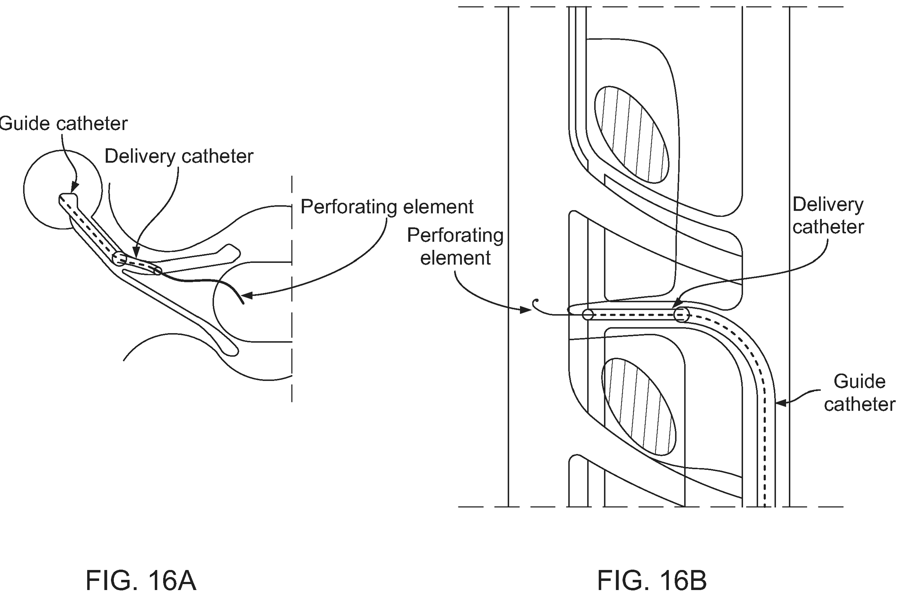

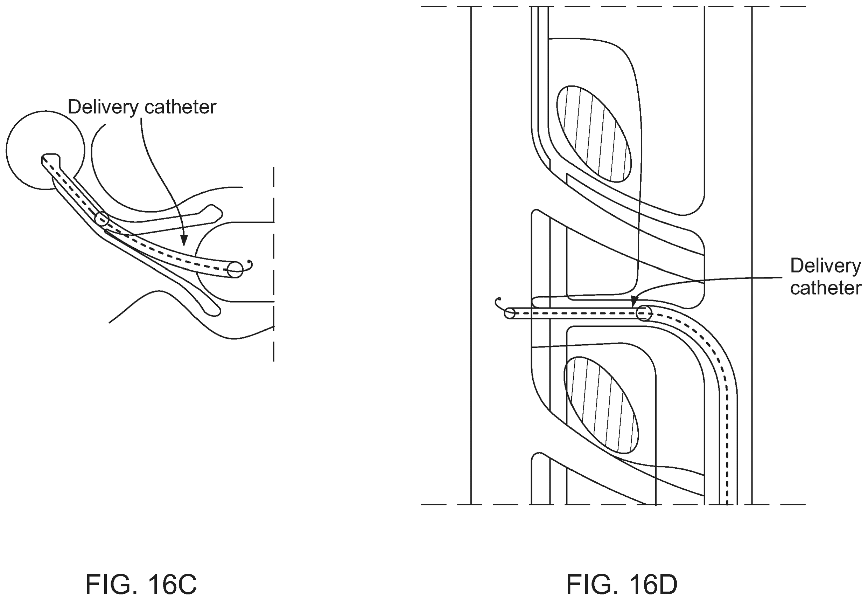

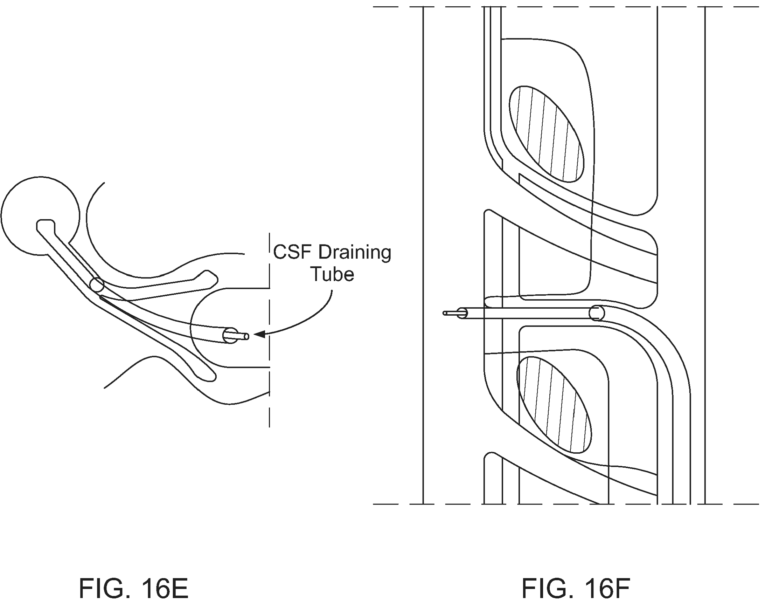

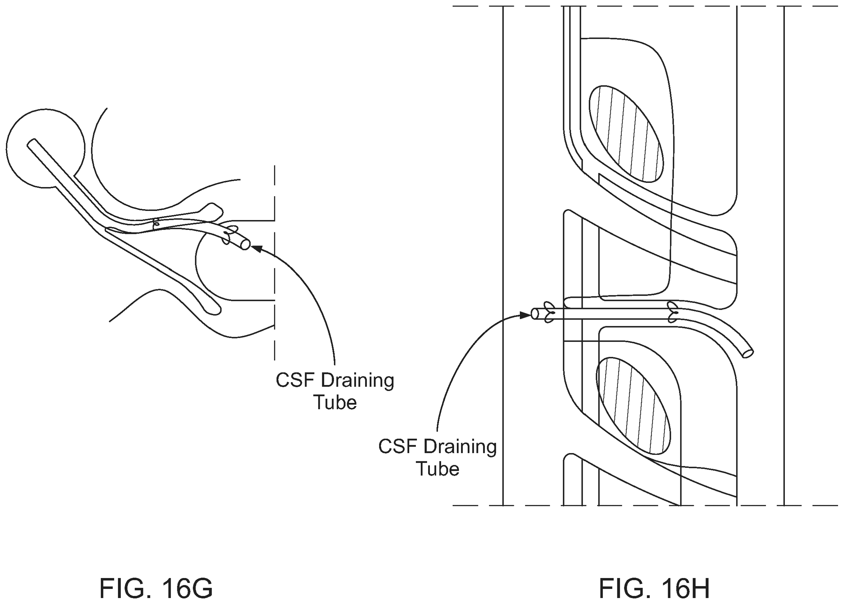

Figs. 16A through 16H illustrate the anatomic approach to accessing the neural foraminal vein with a guide catheter followed by the shunt delivery catheter and dural perforating element. Following the performance of the dural perforating element, the CSF draining tube is delivered creating a communication between the spinal CSF space and venous space.

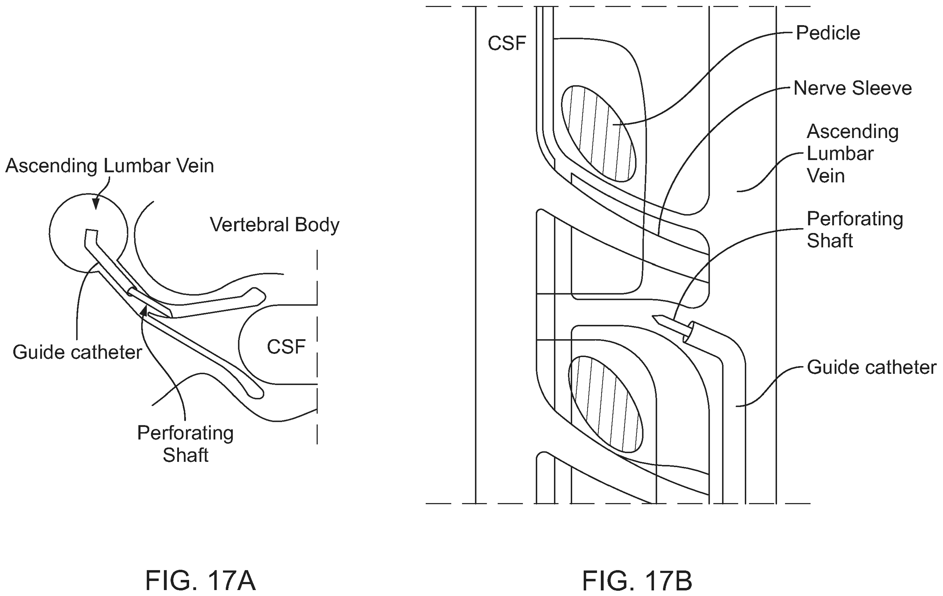

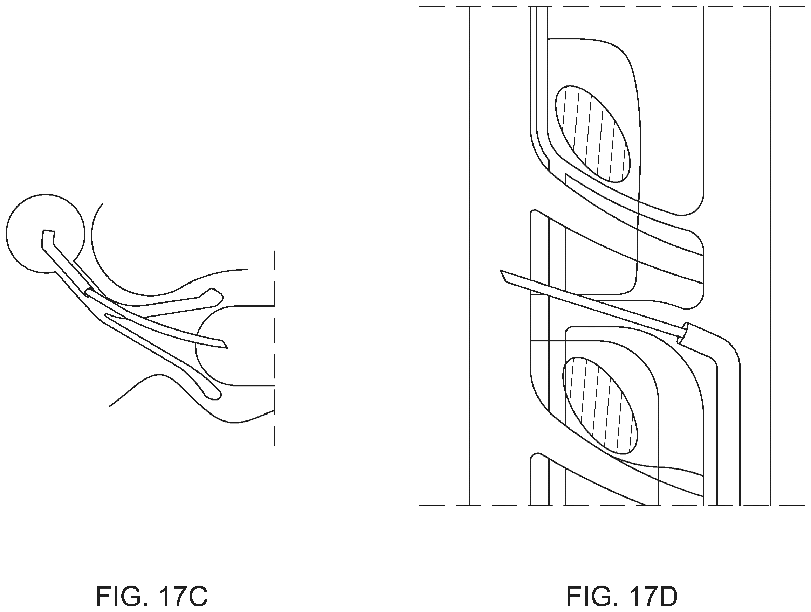

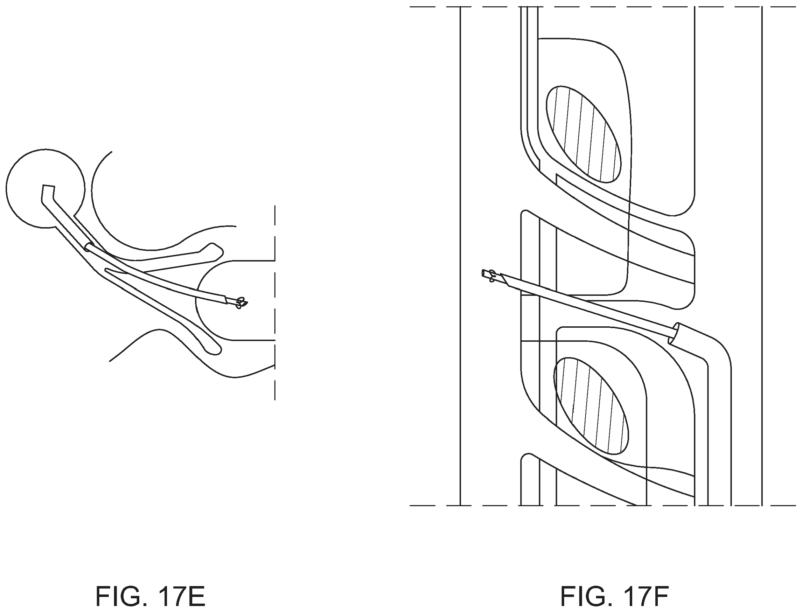

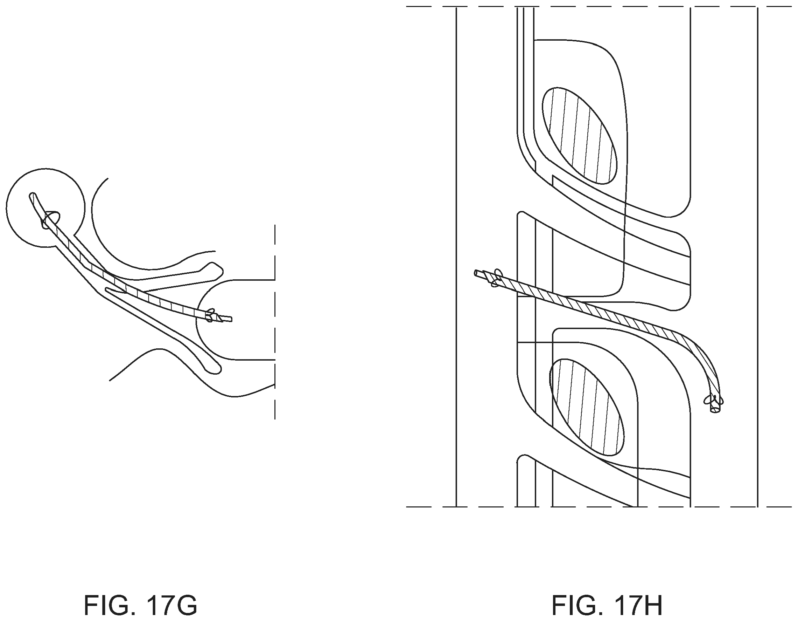

Figs. 17A through 17H illustrate another approach to delivering the shunt catheter. Rather than using electrical or heat energy to perforate the dura, an endovascularly delivered needle hypotube system is used to perforate the dura. The needle can be a straight shape or an S-shape allowing it to deflect off the vertebral body in the ventral epidural space and into the ventral thecal sac. Following this, the CSF shunt is delivered through the hypotube and creates the connection between the spinal CSF space and the venous system.

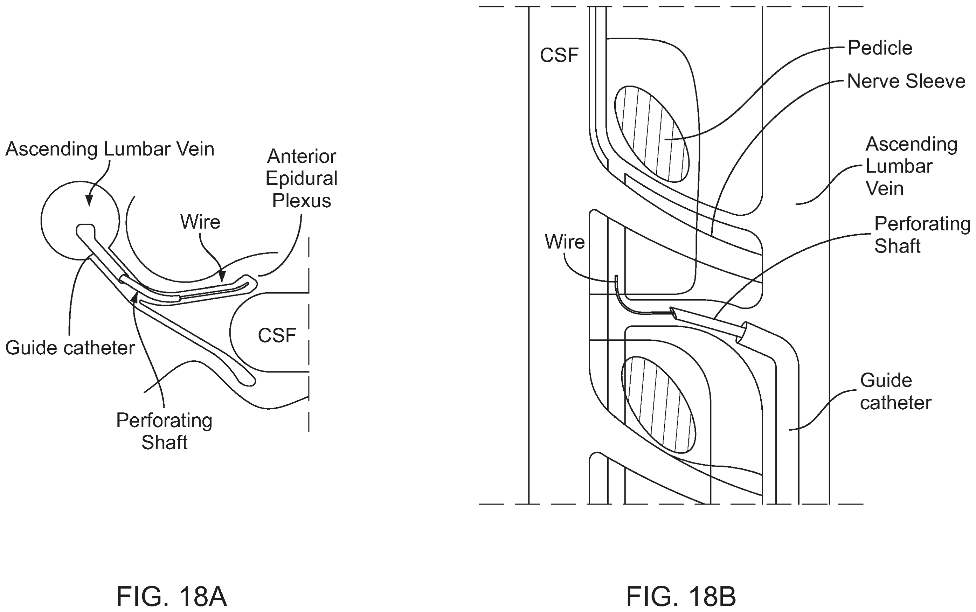

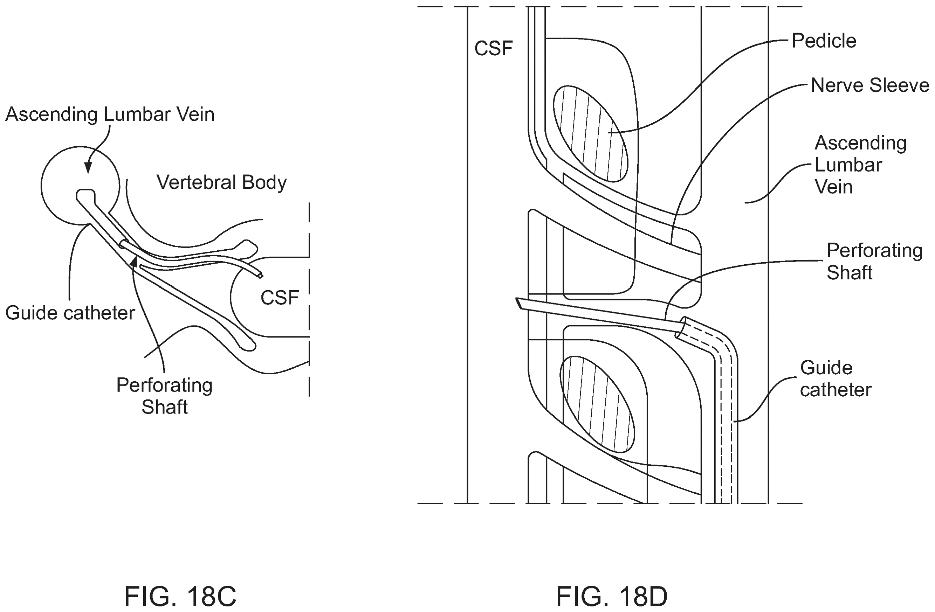

Figs. 18A through 18D illustrate another approach to delivering the shunt catheter whereby a wire is first advanced to the epidural plexus. The needle is delivered over the wire to the desired point of perforation. Once in place the perforating element is used to mechanically perforate the dura thus allowing the shunt catheter to be delivered into the CSF space over the wire or through the hypotube.

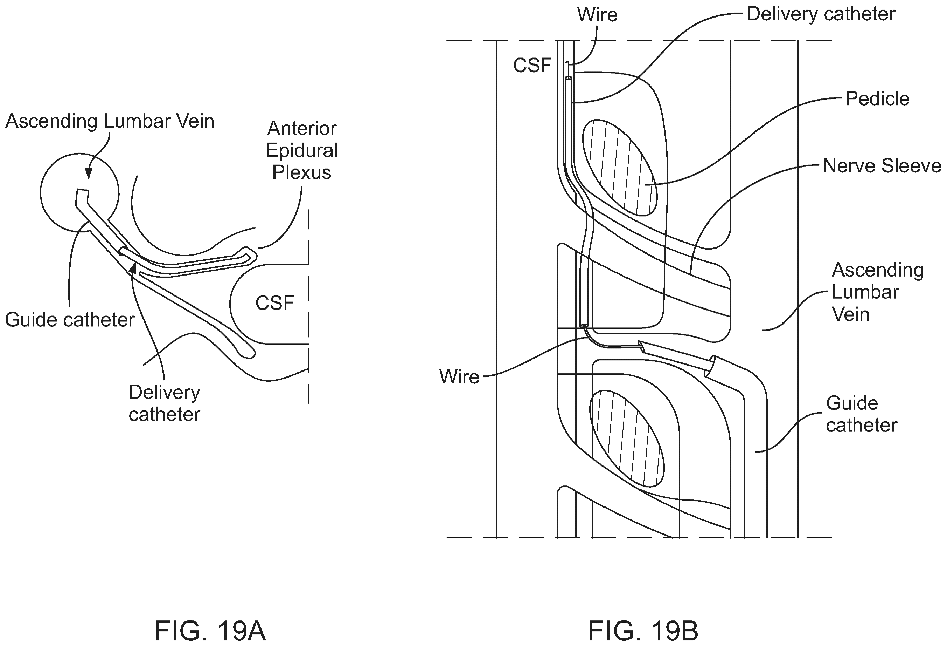

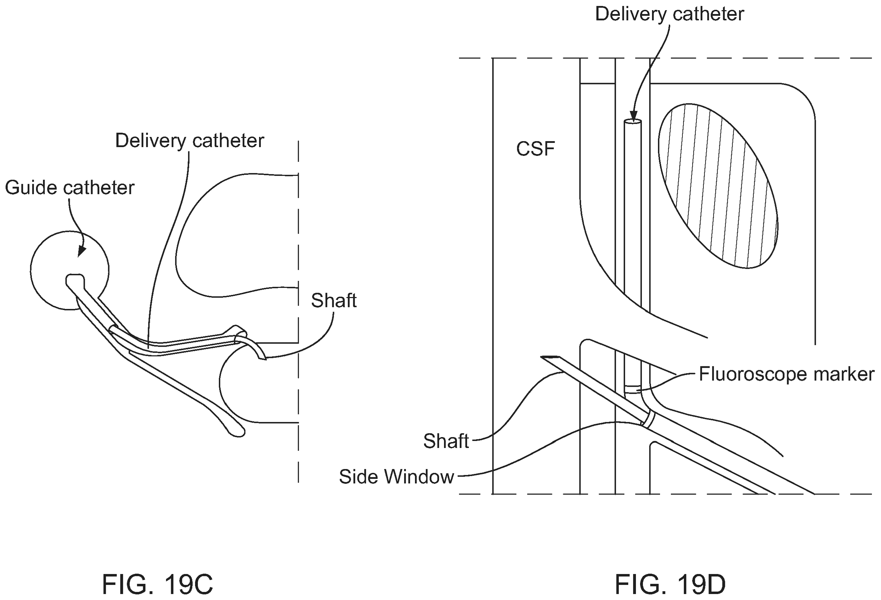

Figs. 19A through 19D illustrate an over-the-wire delivery of the delivery catheter to the epidural vein. The delivery catheter has a hole placed up to 5cm proximal to the tip. The proximal hole in the delivery catheter is directed medially using a fluoroscopic marker which is radiopaque. Then a stiff perforating element is used to perforate the dura allowing for delivery of the wire and drainage catheter.

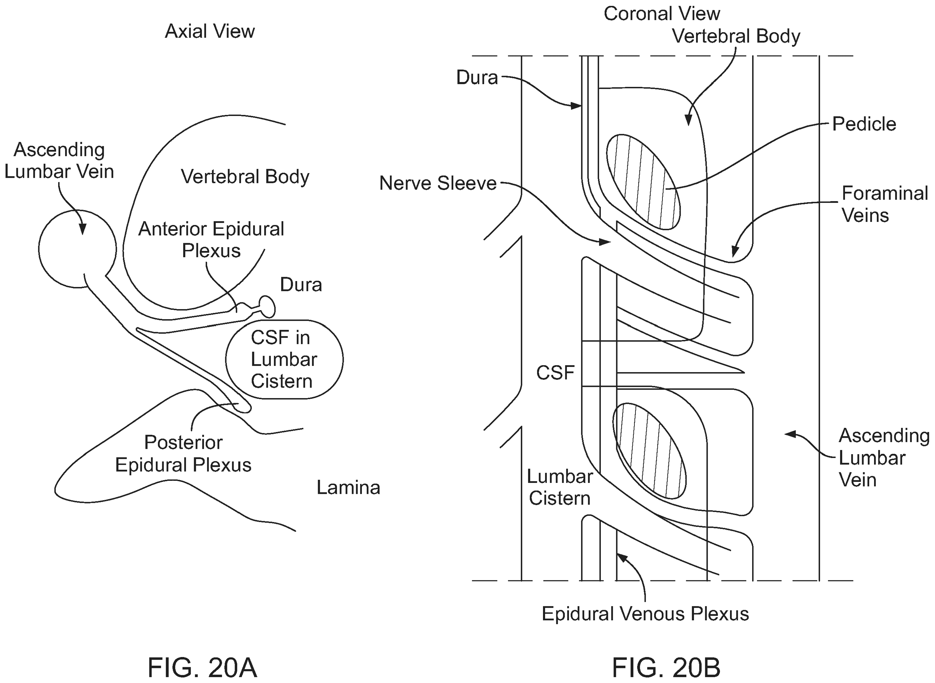

Figs. 20A and 20B illustrate the anatomical configuration of the foraminal veins, anterior and posterior epidural plexus, and their relation to the lumbar CSF space and fluoroscopically visible bone structures including the vertebral body, pedicles and lamina.

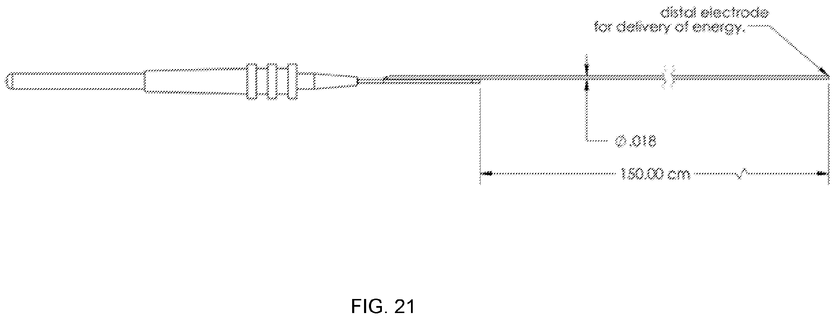

FIG. 21 illustrates an example Bovie knife (which can also be referred to as a Bovie wire) that can be used to perforate, cut, or destroy tissue (e.g., dura) to create a passageway for the delivery and implantation of the catheter shunt devices described herein. The Bovie knife can also serve as a guidewire for deploying a shunt catheter device. In some embodiments, the Bovie knife can be constructed of a 0.018" (0.457 mm, for the conversion 1 inch is equivalent to 25.4 mm) BOVIE LVCSF crossing wire using a ground 304 stainless steel core featuring PTFE insulation for the safety of the user and efficiency of the energy delivered to cross in to the thecal sac. The distal tip of the Bovie knife can include a stainless coil which is soldered or welded to the distal tip of the ground 304 stainless steel core to be the electrode for the delivery of RF energy for tissue perforation and cauterization.

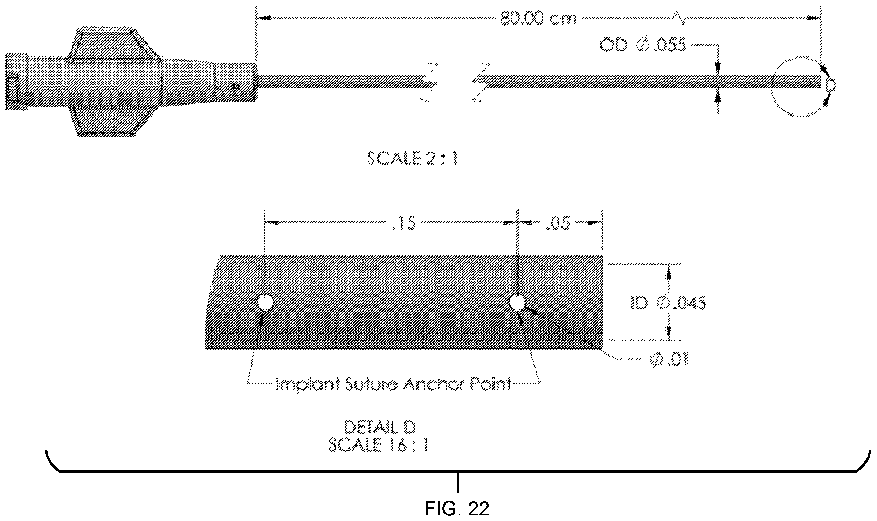

Fig. 22 illustrates an example delivery/push catheter that can be used to advance and deploy a detachable shunt catheter device as described herein. The delivery/push catheter has one or more suture anchor points. A shunt catheter device can be sutured to the distal end of the delivery catheter using a single suture which can be threaded between the four holes on the catheter (e.g., two holes on each side). The suture can be removed to detach and thereby deploy the shunt catheter device from the delivery/push catheter.

In some embodiments, the delivery/push catheter is constructed using a PTFE liner along with 0.002" (0.051 mm) dual start braid with 50 ppi to optimize support and stability during delivery. A 55 durometer pebax can be reflowed on top of the braid to form a thin and pushable shaft. The distal tip can include laser cut implant suture anchor points to connect via suture to the detachable shunt catheter implant devices described herein.

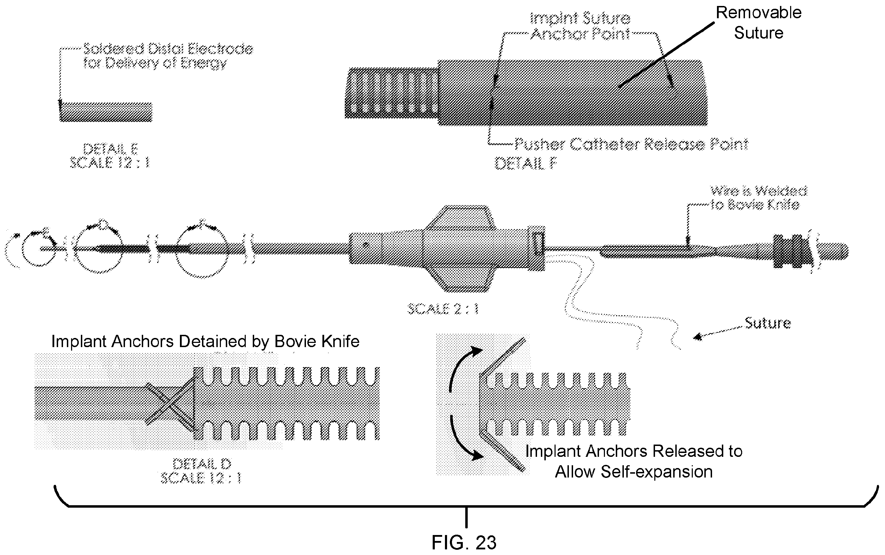

Fig. 23 illustrate the implant suture point of the delivery/push catheter of Fig. 22 and its attachment to the shunt catheter device. Upon removal of the suture (which runs through the lumen of the catheter) the shunt catheter device is released. The Bovie wire runs through the lumen of the delivery/push catheter and the shunt catheter device. Also illustrated are the self-expanding distal nitinol anchors of the shunt catheter device. In some embodiments, the shunt catheter device is constructed of a nitinol hypotube covered in EPTFE.

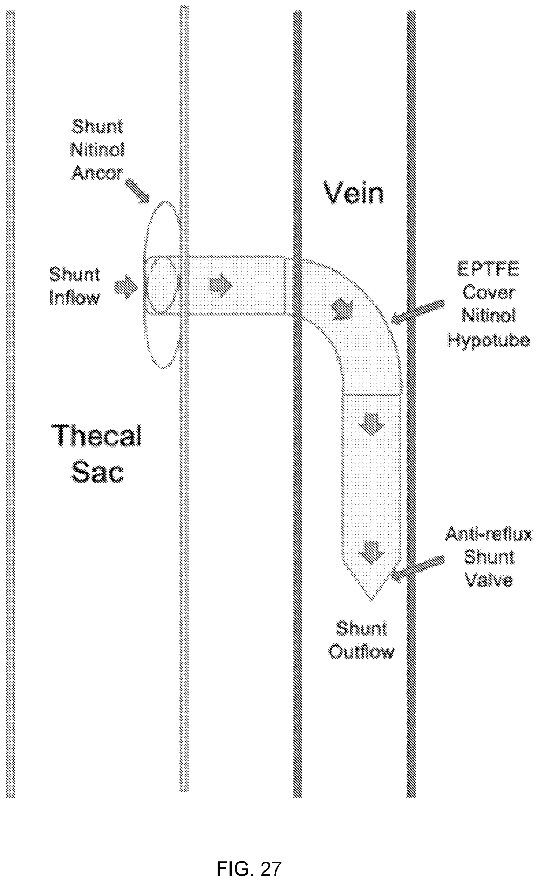

Venus access is gained using a standard access short sheath. A 6Fr angle tip guide catheter can be positioned within the venous system and tracked to the target implant location. Once at the desired target implant location, the Bovie knife/wire is loaded through the detachable shunt catheter device. Both are then inserted into the 6F Angle tip guide catheter. The detachable shunt catheter is connected to the delivery/push catheter with a suture threaded through the pusher catheter release point and shunt catheter suture anchor point. After the Bovie wire/knife has been advanced into the thecal sac with sufficient purchase, the Bovie wire/knife is removed allowing the distal nitinol anchors of the detachable shunt catheter device to deploy radially outward. The suture is then removed to separate the detachable shunt catheter device from the delivery/push catheter-leaving the detachable shunt catheter device implanted and anchored in the anatomy (e.g., as illustrated in FIG. 27).

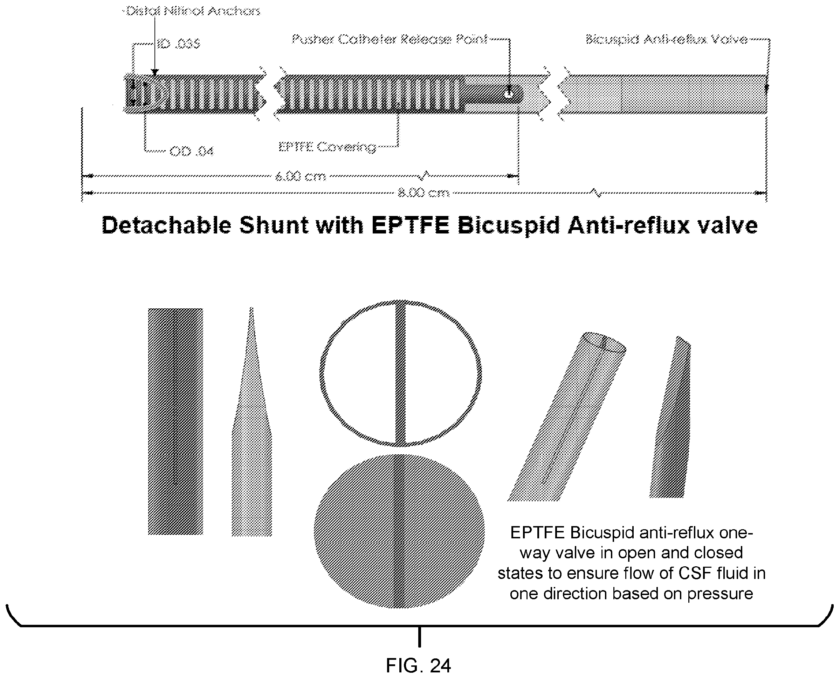

Fig. 24 shows another example shunt catheter device construction. There is a flexible but push-able portion which is placed in the CSF space which is a nitinol hypotube covered with EPTFE. Self-expanding distal Nitinol anchors are present. The proximal end portion contains a bicuspid anti-reflux valve which is a one-way valve preventing reflux of blood into the catheter. This portion becomes positioned in the venous space.

In some embodiments, the detachable shunt catheter device includes an EPTFE bicuspid anti-reflux valve and is constructed using a - 0.003" (0.076 mm) wall Nitinol Hypotube which is cut with a Femto Laser to integrate the distal nitinol anchors and the shunt hypotube in the same low crossing profile. The inner diameter is coated with PTFE to avoid clogging of the shunt hypotube. EPTFE is used to cover the shunt's hypotube outer diameter for smooth delivery and lumen integrity. The EPTFE extends past the proximal edge of the Femto Laser cut nitinol shunt hypotube and is laminated to form a bicuspid one-way valve to ensure the CSF fluid travels in only one direction (e.g., see FIG. 27).

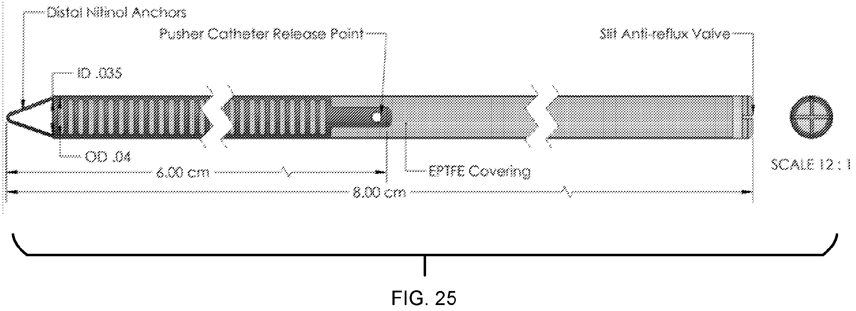

Fig. 25 shows another example design of a shunt catheter device. This shunt catheter device includes a proximal one-way valve that includes a slit valve mechanism. In some embodiments, the shunt catheter device is constructed using a 0.003" (0.076 mm) wall Nitinol Hypotube which is cut with a Femto Laser to integrate the distal nitinol anchors and the shunt hypotube in the same low crossing profile. The laser cut nitinol hypotube is then over-molded with 40A silicone to form a joint less one-piece shunt with integrated slit anti reflux one-way valve. In some embodiments, the entire shunt catheter device (both inner and outer lumens) can be hydrophilic coated to provide smooth delivery and prevent clogging after implantation. The silicone slit anti-reflux valve ensures the CSF fluid travels in one direction (e.g., see FIG. 27).

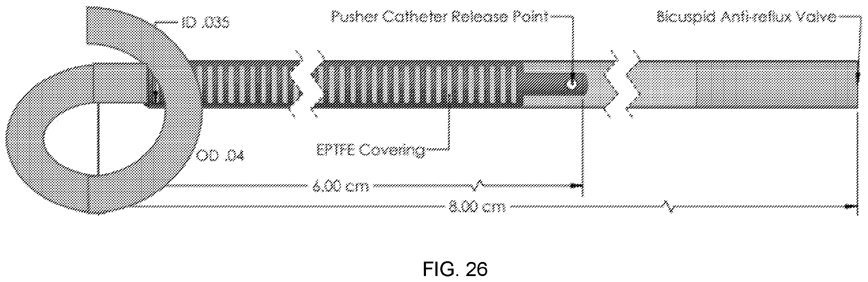

Fig. 26 shows another example design of a shunt catheter device. This shunt catheter device includes distal shape-set pigtail shunt tube (which is naturally curved) to anchor the shunt catheter device in the thecal sac.

Fig. 27 schematically illustrates any of the shunt catheter devices described herein with a distal portion anchored in the thecal sac and shunt tube communicating with the venous system, and allowing flow in only the direction from the thecal sac to the venous system (and not allowing flow in the reverse direction). In some embodiments, the shunt tube includes a natural bend or curve in the mid-body portion of the shunt tube (as illustrated). In some embodiments, the shunt tube is malleable (bendable to various shapes and remains in the bent shape). In some embodiments, the shunt tube is generally linear, but it flexible so that it will readily conform to the patient's anatomy.

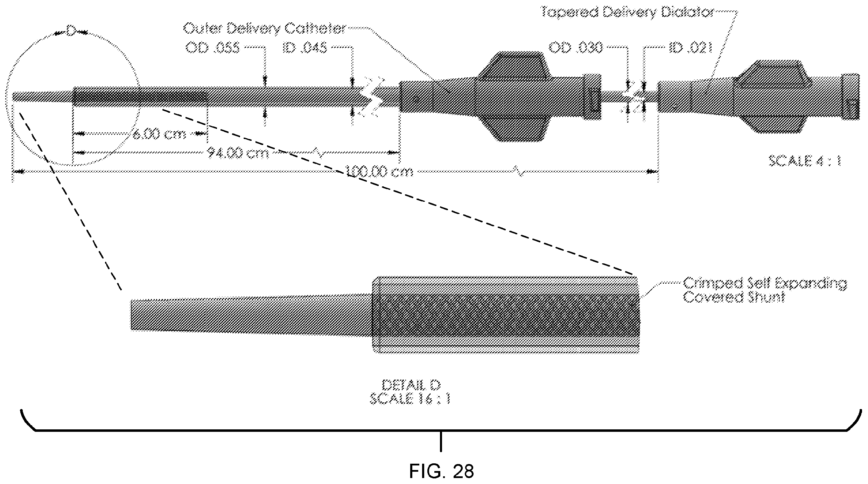

Fig. 28 shows another example system that includes: (i) shunt catheter device as a self-expanding EPTFE covered nitinol hypotube, (ii) an outer delivery catheter, and (iii) a tapered delivery dilator. The shunt catheter device is radially crimped to a lower profile, loaded on the tapered delivery dilator, and placed in the radially constrained configuration within the outer delivery catheter.

The inner tapered delivery dilator and the expandable CSF shunt device are covered by the outer delivery catheter during insertion (except for an exposed distal tip portion of the tapered delivery dilator). Upon the Bovie wire/knife gaining access to the thecal sac, the expandable CSF shunt system depicted is tracked into position over the Bovie wire/knife (to position a distal end portion of the shunt device within the thecal sac). The outer delivery catheter is then retracted allowing the shunt catheter device to radially expand from distal to proximal, with the distal flared end of the shunt catheter device anchoring in the thecal sac. The inner tapered delivery dilator and outer delivery sheath are then removed from the body leaving the expandable CSF shunt catheter device implanted and anchored within anatomy (e.g., as depicted in FIG. 27).

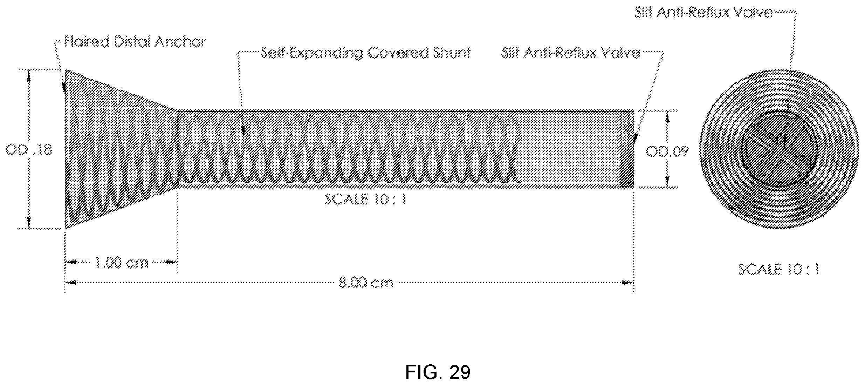

Fig. 29 shows the shunt catheter device of Fig. 28 in a radially expanded configuration. There is a one-way anti-reflux slit valve at the end that becomes positioned in the venous system. There is a flared distal anchor which keeps the shunt catheter device anchored in the CSF space.

In some embodiments, the depicted expandable CSF shunt catheter device includes the EPTFE anti-reflux valve and is constructed by using a Femto laser to cut nitinol similar to existing expandable stents. The nitinol stent frame will then be heat treated to shape-set it into the desired expanded diameter with an flared distal end to anchor in anatomy. The shape-set nitinol stent frame will be covered with EPTFE to allow for sealing against anatomy and for flexibility to expand during delivery. The EPTFE with the shape-set stent frame form the flow path for CSF fluid to flow from the thecal sac into the venous system. The proximal end of the expandable CSF shunt catheter device includes a slit anti-reflux valve to ensure flow of fluid in only one direction (from the thecal sac to the venous system, but not the other way around), relative to pressure differentials.

The present disclosure also covers additional embodiments and variations of the devices, systems, and non-claimed methods described above. For example, in some embodiments a delivery catheter which is typically an elongated tubular element is navigated from a peripheral venous access site (e.g., femoral vein or jugular vein) into the vertebral venous system. In some embodiments, the delivery catheter is angle-tipped or includes a steerable component to gain access to the lumbar vein, usually at an angle of 70-150 degrees (*e.g.,* 90-120 degrees), which is the angle of approach from the ascending lumbar vein to the foraminal veins in Kambin's triangle. The catheter could have sufficient stiffness and torque-ability to orient the curved tip from laterally to medially and direct the perforation element directly towards the dural/thecal sac.

In some embodiments, one or more element can have mechanisms to change the shape, articulate, steer or change the stiffness of at least one segment like pulling micro-wires inside, or a coil pull system.

In some embodiments, the rigidity of a system subcomponent can be modified by air or fluid introduction at variable pressures in accessory channels associated with the wall of these elements.

In some embodiments, at least a segment of one of the elements is deflectable and/or steerable. Deflection (*e.g.,* steering) refers to the movement of the distal catheter segment (*e.g.,* the end) independent of the rest of the catheter. Steerability refers to the ability to rotate or angulate the distal catheter segment (*e.g.*, clockwise and/or counterclockwise with respect to the rest of the catheter) by torque transmission along the length of the device.

The torque causing the deflection can be transmitted by one or more catheters connected to a pull or anchor ring near the device tip. The distal catheter segment rotates one or more directions (*e.g.*, rotational, or flexing within a plane) upon actuation and returns to the original shape (*e.g.,* linear). The deflection can be symmetrical, asymmetrical, loop curves, or compound. Deflection can occur in one or more planes and be on plane and off planes.

For example, in some embodiments, the delivery catheter includes one or more pull wires slidably positioned inside the wall. By pulling on the wires, the distal end segment of the delivery catheter can be laterally deflected. The deflecting capability can also be actuated to anchor (e.g., maintain the position of) the delivery catheter against an internal wall of a vessel and direct the perforation point against the dura and prevent kick back during perforation. The deflecting capability can also be actuated to rest against the bony spinal element during perforation preventing the kick back resulting from the cutting forces required to penetrate dura.

In some embodiments, articulation of the delivery catheter can be achieved with one or more actuators which can provide actuators, such as pull wires, notches, preset curves, shapes, and/or any other mechanism obvious by the ones skilled in the art. In some embodiments, the actuators, such as the pull wires or other articulating elements, are present in the catheter.

In some embodiments, one or more passive (e.g., non-articulable) wires can be used to stabilize and provide direction to the delivery catheter.

In some embodiments, one or more actuators can be attached to the delivery catheter following a pathway, e.g., a linear, curved, "s" shaped, or spiral pathway, along the delivery catheter wall leading to one or more articulation points and/or deflections in one or more directions. This configuration can actively assist in spatially arranging the delivery catheter in an advantageous manner.

For example, in the case of a femoral access approach, the actuator articulates the delivery catheter to form a curve with a posterior and medical concavity from the ascending lumbar vein, resulting in a trajectory pointing towards the spinal dura. If the foraminal vein is the selected exit point, a laterally-to-medially oriented curve will direct the perforating path to the dural sac. If the anterior epidural venous plexus is the selected exit point, an actuator that articulated a proximal segment medially and a distal segment posteriorly will align with the patient of the veins across the foramen into the anterior epidural plexus and direct the catheter tip posteriorly to the dural sac. In some embodiments, these differential curvatures can be obtained by more than one articulating actuator or a spirally-aligned actuator capable of articulating the delivery catheter to curve different segments in different directions.

The delivery catheter can have a pre-made orifice that provides off-axis opening orientable towards the intradural space allowing access to the perforating element. This could be beneficial when the main axis of the vein is about perpendicular to the dura that needs to be penetrated.

In some embodiments, the catheter distal end segment includes a beveled tip, e.g., the opening to the suction catheter includes an ovalized opening and the tip plane can be at an angle with respect to the transverse plane of the suction catheter. In some embodiments, the beveled tip includes a fluoroscopic element for orientation.

The delivery catheter is typically used to deliver a perforating element to the transvascular exit site which is typically a wire or stylet (usually no lumen) or shaft (elongated element with lumen) with cutting or energize-able elements to access the spinal CSF space from the venous intravascular access.

In some embodiments, the distal end of the perforating element includes a cutting feature such as a beveled needle tip, cutting edges, bevels, cutting tips, cone shape, coring punch, and corkscrew shapes. Cutting features can be in the outer edge of the inner edge. The later reverse cutting edge to the inside lumen minimize the gap between two coaxial telescoping elements facilitating perforation at lower forces. Features to facilitate penetration can be one or a combination of multiple features, and it can be combined in any of the device elements including a stylet, shaft or catheter.

The needle tip reduces the applied force to penetrate the vascular wall and dura and decreases the size of the transvascular window decreasing the likelihood of blood extravasation and CSF leakage. A needle can include plurality of cuts to increase flexibility while maintaining column strength as described elsewhere.

Generally, intravascular navigation is facilitated by maintaining the tip of the needle at the front end of the stylet 1 mm distal to the shaft, flush with the shaft, or within 10 mm of the distal end of the shaft. Needles can be larger, the same size, or smaller than the element they are welded on. Minimal gaps (e.g., a diameter difference of less than 0.2 mm) between the telescoping elements reduce the likelihood of catching at the artery/dural wall.

In some embodiments, the perforating element has diathermy, electrocautery or any other electrical feature to facilitate vascular wall perforation, penetration though the extravascular elements (fat tissue, ligaments, joints, etc.) and the spinal dura and entry into the intrathecal spinal space. Diathermy (thermal energy), laser (radiant energy) and electrical energy can also be used to cut and or coagulate intrathecal membranes, septations, arachnoid trabecular and brain and spinal cord tissue (for example the floor of the third ventricle) preventing normal csf flow. Diathermy, laser and electrocautery can also be used to create a CSF-venous fistula, and it can also be used to close the transvascular passageway and the vascular lumen such as the spinal vein. A monopolar or bipolar cautery can be used as a separate component or integrated into the perforating element, the delivery catheter or the drain system or shunt.

In some embodiments, the perforating element can be coupled with thermo-ablation.

In some embodiment, the RF element tip includes two or more electrodes with connecting wires extending from the distal end to the proximal end of the RF element and connected by an electrical joint within a hub to a RF generator. These wires are typically made of conductive metals such as stainless steel, nitinol, copper, and silver and are insulated with plastic layers such as PTFE or by embedding inside the wall of the perforating element, delivery catheter or draining tube/shunt. The electrodes are uninsulated and are made of or coated with conductive and biocompatible metal with high radiopacity such as stainless steel, silver, gold, nitinol or platinum. In some embodiments, one or more electrodes are connected individually to a RF generator to work in parallel in a monopolar manner and share the same grounding pad. In another embodiment with two electrodes, one of the electrodes is connected to the RF generator while the other one of the electrodes is connected to the ground to work in a bipolar manner. In another embodiment, a single or a plurality (>2) of electrodes can be assembled to the RF element and configured to work in monopolar or bipolar manner thereof. In a bipolar system, the current is preferentially concentrated between the two electrodes.

In some embodiments, a bipolar configuration can be obtained by an electrode in the suction catheter and one electrode in the perforating element. The perforating element may acquire a shape upon emergence from the delivery catheter to direct the tip to the venous wall and dura. The penetrating element and the delivery catheter can be concurrently advanced maintaining the distance between electrodes and delivery of current to the tissue, or the perforating element can be advanced while maintaining the catheter stationary resulting in an increased distance between electrode with a drop in tissue disruption and decreased likelihood of unwanted nerve or spinal cord penetration.

In some embodiments, the electrode of the energy delivery device has one of the following shapes: bullet, cone, truncated cone, cylinder, sphere, dome, ring, semi-annular, ellipse, bevel, or arrowhead. The shapes can be at least partially insulated for preferential and current delivery and directional perforation. The electrically exposed area of the electrode is no greater than 16mm², and typically from 1 mm² to 10mm².

In some embodiments, the RF perforating element consists in a substantially tubular member made from an electrically conductive material including stainless steel, copper, titanium and nickel-titanium alloys. The tubular element is proximally coupled to the RF generator and has an electrical insulator disposed thereon to deliver energy to an uninsulated segment or electrode at the distal region with minimal dissipation. The distal end of the tubular element can be open or closed. The tubular element can be tapered, coupled to a hand-held actuator, and can be scored to increase flexibility as described herein. In some embodiments, two or more tubular elements can be coupled to form the RF perforating element. In some elements, a segment of the tubular element (usually the distal most segment) is made from electrically conductive material and electrically coupled to a RF generator. This distal most segment can act as a RF electrode and when coupled with RF energy facilitate transvascular perforation into the spinal CSF space. This embodiment would allow the CSF space to be fluidly coupled with the intravascular space in a manner of a CSF to venous shunt, and the extracorporeal space as a manner of CSF drainage (depending on the length of the tubular element).

Radiofrequency energy is generated by a generator and delivered by one (in a monopolar arrangement) or more (e.g., a plurality) of electrodes (e.g., two electrodes in a bipolar array) attached to the distal end of the penetrating member. In one embodiment, the operating frequency for radiofrequency-assisted perforation ranges from 100 to 1000 kHz, typically between 300 to 600 kHz. Typically, high impedance (1500 and 5000 Ω) results in smooth penetrations with minimal spreading damage in surrounding tissues. The typical generator power for venous wall and dura perforation is 3 watts to 30 watts. The voltage to facilitate RF perforation rages between 75 and 1000V, and it is typically between is 120V to 400V. Typically, these conditions allow perforation from the intraluminal space into the intradural spinal space with activation time <10 seconds. Duty cycles can be in pulses of 5ms to 1second, typically around 200ms to 500ms. The electrical waveform generated by the generator can be sinusoidal and rectangular, amongst others. Typically, RF perforation is concurrent to forward pressure transmitted through the apparatus from the proximal end.

The electrical source can be integrated to the device, or it can be delivered by contacting a part of the device outside the patient in electrical coupling with the perforation tip with a Bovi electrocautery knife/wire or similar generator.

In some embodiments, a heat shield formed by thermally insulative material like Zirconium Oxide or PTFE can be applied adjacent to the electrode.

In other embodiments, different energy sources may be used to facilitate perforation including laser, microwave and ultrasound.

In some embodiments, the RF wire acquires one or more curves equal or higher than 1 degree or a pigtail shape upon emergence from the delivery catheter. This may be beneficial to direct the perforation to the dura, prevent unintentional pullback into the vascular lumen and to prevent spinal cord perforation during device advancement.

In some embodiments, the penetrating element includes one or more apertures in the distal segment fluidly coupled to channels to inject contrast through the injection port to confirm the perforation of the targeted tissue, saline solution to cool the surrounding tissue to reduce the thermally affected zone during radiofrequency perforation, to measure pressure changes or enabling csf emergence indicating entry to the intrathecal spinal space.

In another embodiment, one or more thermocouples are attached near the distal end of the penetrating member to monitor the tissue temperature at and/or near the targeted site.

In another embodiment, the penetrating member includes one or more sharp edges, such as a bevel, and perforates the vessels, extravascular tissues and dura which reduces the RF energy to perform the penetration. In alternative embodiments, the RF wire has an atraumatic non-cutting tip.

In some embodiments, the RF element, which can be the wire, catheter, catheter, or the combination of components thereof, includes a combination of electrodes, temperature sensor, and pressure sensor.

In some embodiments, self-orientation is achieved by pre-shaping wires, tubes or other elongated element made of elastic material (for example nitinol) with one or more curves. The flexible nature of this material allows to be advanced inside catheters, and spontaneously rotate in space to best align the pre-set shape to the 3D venous vascular anatomy. Leveraging the 3D anatomy of the veins at the foramen and epidural plexus, the orienting shape of the elongated material can be coupled with additional distal curvatures to direct the perforating element to the dural sac. In these embodiments, the hard tissues of the spine (like bones and articulations) behave like levers that induce rotational motion to the elongated element while the latter releases the elastic energy while becoming unconstrained and approximating to the pre-set shape. For example, if the anterior epidural venous plexus is the selected exit point, the pre-shaped perforating element may have a medially directed curve to orient at the foramen in a laterally-to-medially direction, a more distal curvature anteriorly to enter the anterior epidural plexus, and a distal-most posteriorly oriented curvature to direct the front end of the perforating element to the posteriorly located dural sac. The penetrating element can be electrically coupled to RF generator.

In some embodiments, insufflating a balloon, deploying a stent, or other expandable element disposed in a proximal or distal vascular segment and mechanically connected to one or more of the telescoping elements can enhanced pushability or act as distal anchors of the perforating element and decrease kickback.

In some embodiments, enhanced pushability of the perforating element, decrease the kickback of the delivery catheter and directionality can be achieved by advancing over a wire a delivery sheath with a branching annex into an arterial bifurcation. A catheter including a second catheter can include an annex to stabilize the system and provides 3-dimensional orientation to perforate the vascular wall and dura.

In some embodiments, the perforating element is a needle that is advanced though the catheter to penetrate into the intradural space. The shape of the perforating element can be permanent or composed of a malleable material (e.g., nitinol).

In some embodiments, the perforating element is hollow and a contrast agent can be injected into the intradural space though the perforating element lumen.

The shape can be also provided by a shaped inner element within the perforating element. The distance that the perforating element is advanced beyond the suction catheter or delivery sheath is called the perforation distance (dₚ). This distance, dₚ, can be fixed, temporarily fixed or adjustable.

A typical depth of penetration includes a range from 1 mm to 20 mm (e.g., from 10 to 20, from 2 mm to 10 mm, 4 mm to 10 mm, 60 mm to 10 mm, 8 mm to 10 mm, from 1 mm to 8 mm, from 1 mm to 6 mm, from 1 mm to 4 mm, or from 1 mm to 2 mm). In some embodiments, the penetrating element is used to create the transvascular corridor and penetrate through tissues, and also used for long distance navigations in the intradural space. For example, a RF tipped wire can be energized to cut through the vascular wall and dura, and then used without energy to atraumatically advance in the CSF space.

The depth of penetration is adjustable based on, in some examples, images obtained before or during the intervention, such as CT scan image and/or spine MRI image. The wire and/or catheter advances into the intradural space into a final position and the handle operated to lock the catheter position. The wire is removed and contrast injected into the intradural space. The area is imaged using a non-claimed method described herein to confirm the intradural location of the catheter distal end. A wire is advanced coaxially into the catheter into the intradural space.

In some embodiments, the perforation element can be the wire, a shaft, the delivery catheter or the CSF draining tube.

In some embodiments, the telescoping elements includes one or more structural elements (e.g., rails) to increase the element stiffness, minimize unwanted rotation, and maintain the orientation along the plane. The railing system may have at least a portion of the length one of the following cross-sectional shapes: circular, oval, square, start, diamond, rectangular, flat, or a combination thereof. In a telescoping embodiment, the receiving lumen may conform to the shape of the inner member.

Non-circular configurations of the wire, shaft and catheter can limit the relative rotation between subcomponents while maintaining the capacity to telescope along the longitudinal axis (e.g., longitudinally). Such non-circular embodiments help to maintain the trajectory of the perforating element over the guide element towards the perforation target. As one example, the rail system may be beneficial when advancing the penetrating element over a distal anchoring element. In this example, the anchoring element (balloon or stent or other) can be connected to the device by a flat wire. The flat wire can allow element advancement over the wire of the perforating element (or protective sheath) to the perforation point. In other embodiments, radial alignment between telescoping elements can be maintained by coupling longitudinal recesses and fins at the interfacing surfaces.

In some embodiments, one or more subcomponents include one or more limiting features to limit the penetration depth to within a distance of the venous wall and/or dura perforation. These embodiments could be especially beneficial at the occipito-cervical junction, the cervical and thoracic levels where the spinal cord is typically 1mm to 10mm of distance from the dura. Venous wall are significantly weaker than the dura and these limiting features can be design to differentially control penetration depth. The limiting features include elements that expand and/or deploy resulting in a larger dimeter than the subcomponent carrying these elements (or alternatively expand the subcomponent diameter), and examples of arresting features include barbs, fins, wires, collar, rib, rim, ribbon, and baskets. In some embodiments, the limiting features are recaptured (e.g., retracted). Other limiting feature examples can be elements that reduce the subcomponent diameter along a portion of the subcomponent length such reversible bevels, indentations, or notches. In some embodiments, the limiting features are manually or automatically retractable (e.g., hide-able).

In some embodiments, the perforating element can have fixed features to limit the penetration depth. This is beneficial when the perforating element is not intended to advance beyond a point but enable an inner element and or an outer element to be advanced distally. As an example, a beveled cutting shaft (elongated tubular element) can be the penetrating element that pierces the venous wall and dura until and advances into the intradural space and is stopped by a rim. The rim can be sufficiently sized to push the venous wall and improve apposition with the dura during dura perforation by the beveled tip. After perforation, a micro-wire is advanced inside the shaft and distally into the intradural space. Then, the CSF draining tube is advanced over the shaft though the vessel wall and over the micro-wire though the intradural space while the fixed shaft is providing column support to the remaining advancing elements. In some embodiments, the CSF draining tube is partially advanced distally to the shaft front end aperture into the CSF space. The shaft is then pulled back resulting in unsheathing of the CSF draining tube from the intradural CSF space, through the extradural extravascular space into the intravascular venous space.

In some embodiments, the perforating element that initially pierces the dura has features that deploy in the unconstrained CSF space to prevent nerve roots and spinal cord damage.

In some embodiments, one or more device subcomponents could have one or more features to minimize the risk of unintentional pull back into the artery after perforation and to limit the longitudinal advancement beyond a certain point.

In other embodiments, a protective sheath can be added to the telescoping system and be disposed over the perforating subcomponent. The protective sheath can be disposed throughout the length of the perforating subcomponent or be selectively disposed to cover the distal segment of the perforating component. In such cases, the protective sheath is translated longitudinally by one or more pull or push wires. The protective sheath protects the cutting features of the perforating element and the inner surface of the delivery catheter during the advancement of the perforating element to the target. The protective sheath provides orientation for the inner perforating element when the protective sheath is coupled to the anchoring element (balloon, stent, etc.) by a rail system as described herein. The protective sheath enables a perforation element to acquire a memory shape when unsheathed from the protective sheath. The protective sheath can have one or more fluoroscopic elements to indicate the location of the perforating element.

In other embodiments, the shaft is constructed with perforating elements described elsewhere herein and actuated to perforate the venous wall and dura without an inner perforating wire (or stylet). The shaft can have a lumen to inject a radio-opaque contrast agent to ensure that the tip opens to the spinal CSF space. The shaft lumen could also be used to infuse saline which would flow with low resistance if injected in the CSF space, and at a higher resistance if injected into the extravascular epidural space. An atraumatic stylet or micro-wire can be coaxially introduced inside and beyond to the shaft into the subdural space. The delivery catheter can be advanced over the shaft though the vascular wall and the dura, and then over the stylet/micro-wire inside the intradural space.

Any of the elements herein described can be telescoped co-axially and/or over the wire rapid exchange system.

In some embodiments, the device includes an imaging component. For example, the device can include an optical coherence tomography (OCT) or an intravascular ultrasound (IVUS) component.

In some embodiments, the perforation process and ingress in the csf space can be monitored by including one or more sensors in the device, including pressure sensors, e.g., potentiometric pressure sensors, inductive pressure sensors, capacitive pressure sensors, strain gauge pressure sensors, fiber optic pressure sensor, variable reluctance pressure sensors, microelectromechanical system pressure, and piezoelectric pressure sensors.

In some embodiments, the perforation process and ingress in the CSF space is monitored by measuring impedance or permittivity of tissue and fluids.

In some embodiments, the perforation process and ingress in the intradural space is monitored by including a force sensor at the proximal end of the perforating element to measure a thrust force associated with penetration through the vascular wall and dura.

In another embodiment, the perforation process is followed by measuring pressures at the tip of the perforating element by channel fluidly coupled to a pressure transducer which can be outside the patient.

In another embodiment, the perforation process is followed by coupling the wire, catheter or delivery catheter with an electrode for nerve sensing and or nerve stimulation. Typically, the stimulation is done from 0.01 to 20mA stimuli (usually <5mA). Nerve and nerve root stimulation enables mapping of the nerve through the tissue in the foramen, lateral recess and intradurally. The sensing (recording of resting potentials and compound action potentials) and or stimulation electrode can function as the RF energy delivery device. In some embodiments, an interface unit working as a splitter enables concurrent connection of the apparatus to a RF generator and nerve sensing and or stimulation.

### SHUNT

In some embodiments, the system includes a tubular element design to fluidly couple the spinal CSF space with the intravascular space in a manner of a CSF-venous shunt, and/or the spinal CSF space to the extracorporeal space as a manner of CSF drainage. Herein this element is called the CSF draining tube.

In some embodiments, the CSF draining tube can be advanced over a wire across the trans-vascular space into the spinal CSF space. The wire may have been the perforating element or was introduced transvascularly into the spinal CSF space though a perforating element. In other embodiments, the CSF draining tube is delivered (pushed out, unsheathed, or a combination) through the lumen of a delivery catheter already contacting the spinal CSF space.

In some embodiments, the CSF draining tube is formed by more than one segments that can be detached or uncoupled (mechanically, electrolytically, electrical, chemically, retention sleeves, pressure-operated systems, rotational system, fracturable elements, detachment actuator, etc). Detachment of the distal segment of the CSF draining tube can convert a CSF draining catheter (extracorporeal) to a CSF-venous shunt. If CSF drainage or diversion is no longer required or beneficial, the whole CSF draining tube can be removed by percutaneously pulling the un-detached system. This embodiment would be highly beneficial in situations requiring an initial period of external CSF drainage, and then, in a subset of patients, internal CSF diversion in the form of a CSF-Venous shunt. Clinical conditions that will benefit from the optionality to completely remove the system vs implanting a long-term CSF-venous shunt include subarachnoid hemorrhage and normal pressure hydrocephalus.

In some embodiments, the CSF draining tube has one or more anchoring features to anchor the CSF draining tube to the intradural surface after penetration. Examples of these anchoring features include wires (including shaped wires, e.g., pigtail wires), stents, balloons, arrowheads, wings, fins, loop, bend, harpoons, spikes, hooks, and/or barbs. In some embodiments, these anchoring features may be compressible (e.g., formed of a low durometer material) to be recaptured, deployable/recapturable, unsheathable/resheathable.

In some embodiments, the anchoring features are actuatable to anchor the CSF draining tube upon actuation or activation. Alternatively, the anchoring features are fixed (e.g., static) to provide anchoring coincident upon ingress into the CSF compartment.

In some embodiments, the penetrating element can become the anchor upon actuation or advancement. As an example, the penetrating wire can be used to penetrate through the vascular wall and dura, and upon emergence from an enclosing subcomponent acquires a pigtail shape which coincidently anchors the wire in the subdural space. The wire pigtail shape provides an atraumatic tip upon advancement in the subdural space.

In some embodiments, one or more subcomponents of the system have one or more arresting features which minimize the risk of unintentional subcomponent retreat into the epidural space or intravenous space after perforation of the venous wall or dura. This is advantageous as un-intentional retreat may result in bleeding though the venotomy. The arresting features include elements that expand and/or deploy resulting in a larger dimeter than the subcomponent carrying these elements (or alternatively expand the subcomponent diameter), and examples of arresting features include barbs, fins, wires, collar, rib, rim, ribbon, deployable covered stents, and baskets. In some embodiments, the arresting features are recaptured (e.g., retracted). Other limiting feature examples can be elements that reduce the subcomponent diameter along a portion of the subcomponent length such reversible bevels, indentations, or notches. In some embodiments, the arresting features are manually or automatically retractable (e.g., hide-able).

In some embodiments, the tip end of the CSF draining tube can be closed and/or have different shapes. The CSF draining tube can have side holes/fenestrations to facilitate fluid flow. In some embodiments, the CSF draining tube end is tapering and has no opening at the tip. This design has no ledge and therefore is highly atraumatic. Some embodiments may have an opening at the end to allow coaxial advancement of a shaft disposed at least partially inside the CSF draining tube.

In some embodiments, the distal tip of the CSF draining tube can have bevel and sharp edges to facilitate the entry into the intradural space and element in the wall to prevent penetration into the spinal cord or nerve roots. CSF draining tube with an oval lumen rather than round can decrease likelihood of neural tissue penetration. CSF draining tube with beveled tip can facilitate drainage of fluid and clots.

In some embodiments, CSF draining tube can have a distal funnel to enhance drainage of fluid and clots. The funnel can be open by a balloon system, pull or push wires, or have a braided design or slotted hypotube design that can be introduced in a compressed state and then self-expands into a funnel after unsheathing. In some embodiments, similar designed can be employed into a balloon-mounted of self-expandable covered-stent to maximize the lumen of the CSF draining tube while anchoring across the durotomy and into the recipient vein.

The lumen of the CSF draining tube can be configured to enable delivery of devices and irrigation of solutions, drugs, cells, or particles. In some embodiments, the CSF draining tube may have additional hollow channels to enable fluid irrigation in the setting of hematoma. The fluid can be directed outside, at the tip or inside the CSF draining tube.

In some embodiments, the fluid can deliver pharmacological agents to the treatment location, or suspended cells and particles in a solution which is later aspirated by the suction catheter.

In some embodiments, the lumen of the CSF draining tube can be coated by lubricious substances to facilitate drainage of fluid and particulate matter.

In some embodiments, the CSF draining tube can be coupled with a pump for intrathecal drug delivery.

In some embodiments, the CSF draining tube can be coupled with a reservoir amenable to percutaneous access for CSF aspiration and/or contrast, drug, biologics, cells, diagnostic or therapeutic agent injection.

In some embodiments, the CSF draining tube can be coupled to a vacuum source. The applied vacuum can be continuous, dynamic, cyclical, pulsatile, at low and/or high frequency. In the case of subarachnoid hemorrhage, pulsatile pressure can be beneficial to induce clot fatigue and fracture facilitating aspiration removal. Fluid drainage can occur spontaneously by a pressure gradient between the intraspinal compartment and the atmosphere. Vacuum can be applied either using syringes or pump.

In some embodiments, the CSF draining tube has pressure valves which open at a preset pressure gradients, typically 1 mmHg to 20 mmHg (133 to 2666 Pa). In some embodiments, the pressure gradient of valves can be adjusted including by magnetic field, electric fields, microelectromechanical systems.

In some embodiments, the CSF draining tube has one or more slits on the intravascular segment.

In some embodiments, the CSF draining tube has one more valves for unidirectional CSF drainage (to the veins or outside the body) and preventing blood to reflux into the spinal CSF space.

In some embodiments, the CSF draining tube has mechanisms to regulate the flow.

In some embodiments, the lumen and length of the CSF drain tube result in the resistance required to enable drainage of CSF in similar amount than the physiological production, typically 15-30 ml/hour.

In some embodiments, the lumen of the CSF drain tube is sized 0.005" to 0.025" (0.127 to 0.635 mm), typically 0.010" to 0,020" (0.254 to 0.508 mm), (typically 0.010" to 0.020") to facilitate low resistance and high velocity to prevent thrombosis and plugging.

In some embodiments, at least a segment of the CSF drain tube is coated with substances to reduce thrombogenicity, for example carbocel heparin.

The construction of the wire, delivery catheter, CSF draining tube or catheter to achieve these features comprises one longitudinal element selected from the group consisting of a hypotube, single solid rod, multiple rods, bundle, tubing (with one or more lumens), shaft strands, cable (two or more wires running side by side, bonded, twisted or braided), coil, braid or combinations thereof.

In some embodiments, the device subcomponents can be made of metal or metal alloy (including but not limited to stainless steel, nitinol, silver, titanium, copper, cobalt chromium, nickel chromium, platinum iridium, and others), polymer (including but not limited to nylon or other polyamides, fluoropolymers, polyolefins, polythetrafluoroethylene, high density polyethyene, polyurethanes and polyimides), ceramic, bio-absorbable or dissolvable components, or combinations.

The construction of the device can include inner liners and outer jackets and the manufacturing techniques are known by those skilled in the art. These elements may be necessary to enhance the structural support to the device, facilitate smooth telescoping between components, prevent vacuum leaks by sealing holes, and enhancing the flow of hematoma to be drained and reduce the likelihood of clots and fibrin to clog the suction catheter.

It is beneficial for the elements of at least some of the devices described herein to remain highly flexible to navigate into the spinal intrathecal compartment and have sufficient column strength to perforate and advance into the intradural space. To this end, the elements have sections of varying stiffness. This is accomplished by employing and combining different element construction configurations, materials, ration of materials, thicknesses, amount of material, materials with different durometer, and/or selective reinforcement.

In some embodiments, any of the subcomponents may include a plurarity of scorings to increase element flexibility, for example, to transverse the curves of the foramen. The plurality of scorings can take a shape or pattern including but not limited to spiral scoring patterns (continuous or interrupted), radial scoring patterns, bespoke scoring patterns, radial ring patterns, longitudinal scoring, oblique scorings, windows, tabs, or holes. Scorings may be created in the elements by using any suitable scoring methods including laser scoring and etching. Scorings can be in at least a portion (e.g., a segment) of the subcomponent and in some embodiments are preferentially located on one side.

In some embodiments, any of the subcomponents have one of the following profiles along its length: continuous, tapered in distal direction, tapered in proximal direction, multi tapered and combinations thereof.

Generally, subcomponents are larger, stiffer, and have higher torque transmission proximally and will taper distally and have increasing flexibility to enter the spinal foramen.

In some embodiments, any subcomponent, is constructed with two or more layers of high-tensile wire wound at opposing pitch angles resulting in flexible elements with high torsional stiffness.

Fluoroscopic elements (e.g., radiopaque markers) that are highly visible under fluoroscopy can be located on any of the components in any locations as desired.

In some embodiments, the fluoroscopic elements construction can include elements indicating the direction of the deflecting element and/or the pre-shaped elements. The direction of the deflecting element indicates the plane to which the marker will direct the subcomponent, e.g., the plane of deflection. For example, the marker can be a partial circle with a notch matching the plane of deflection, or the plane of deflection can be marked by the partial radio-opaque circle, or by an asymmetry in the marker. Alternative examples of fluoroscopic elements include an arrow-head, ring, or band symbol or structure.

In some embodiments, the fluoroscopic elements provide support to the perforating element in a circumferential fashion.

In some embodiments, the fluoroscopic elements decrease the cutting force required to perforate the MMA and/or dura, such as a fluoroscopic marker including a beveled tip, or cutting tip.

In some embodiments, the fluoroscopic elements can be tapered to decrease the gap with the OD of a telescoping element.

In some embodiments, two or more fluoroscopic elements are present in a subcomponent. For example, the delivery catheter or the CSF draining catheter can have one fluoroscopic elements distally to indicate the distal end of the device, and another fluoroscopic elements in a more proximal segment to indicate the detachment of the element to close the arteriotomy, including off-the-shelf coils.

In some embodiments, two or more different subcomponents have two or more fluoroscopic elements which indicate longitudinal and or rotational alignment between subcomponents. These fluoroscopic elements can include symbols (e.g., "bullseye"), forming intersecting shapes (e.g., "T", "L","+", or "X"), or other way evident by those skilled in the art.

In some embodiment, one or more magnetic component can be added to any component of the system for magnetic-based movement.

In some embodiments, the CSF draining tube is removed and the durotomy and/or venotomy is closed by one or a combination of the following hemostatic mechanisms: balloon, gel foam, collagen, thrombin, particles (e.g., polyvinyl alcohol, embospheres), coils (e.g., pushable, injectables, detachable), liquid agents (e.g., glue, ethylene vinyl alcohol), sclerosant agents (e.g., sodium teradecyl sulfate, alcohol, algel), plugs (e.g., including self-expandable cylindrical or hourglass shape), stitches, electrocoagulation, radiofrequency energy.

In some embodiments, the venotomy is very small and would not result is significant blood extravasation making the hemostatic device not needed.

***Foraminal Access (Lumbar Vein Approach).*** In a preferred embodiment, the ascending lumbar vein on the left would be the used to navigate a catheter system into the spinal venous system. To this end, the left common femoral vein access would be obtained. A catheter would be advanced into the left ascending lumbar vein. The catheter could be angle tipped or contain a steerable component to allow it to access the lumbar vein, usually at an angle of 70-150 degrees (typically 90-120 degree) which is the angle of approach from the ascending lumbar vein to the foraminal veins in Kambin's triangle. The catheter could have sufficient stiffness and torcability to orient the curved tip from laterally to medially and direct the perforation element directly towards the dural sac.

Following this, perforation of the dura is performed using an RF tipped wire which extents distal to the tip of the delivery micro-catheter (3-4Fr) and delivers an RF pulse. In some embodiments, the catheter is designed to advance over a wire into the CSF space. In this embodiment, there is minimal step off between the RF tipped wire and the tip of the catheter to allow for the whole system to enter the thecal sac with one push. In other embodiments, the delivery catheter is designed to remain in the intravascular space, and allow the delivery of an elongated element that will trans-vascular advance co-axially over the RF tipped wire into the CSF space.

In some embodiments, the RF wire could be insulated except the tip. In some embodiments, the RF tipped wire is temperature controlled. Temperature may be gradually increased from 45-65 degrees centigrade and the patient will be monitored to ensure that there is no nerve stimulation or pain. If no nerve stimulation or pain, the temperature is increased to allow for penetration of the dura mater. Once the wire is advanced into the thecal sac, confirmation of positioning is performed by withdrawing the wire and injecting contrast through the catheter.

In some embodiments, the RF tipped wire includes a stimulation mode and a cauterization mode. At the stimulation mode, electricity is delivered and the patient monitored to evaluate for nerve stimulation or pain. If no nerve stimulation or pain, the RF tipped wire delivers RF energy for penetration of the dura mater. Once the wire is advanced into the thecal sac, confirmation of positioning is performed by withdrawing the wire and injecting contrast through the shaft or catheter.

***Epidural Access.*** In this location, the trajectory of penetration is about perpendicular to the main axis of the venous plexus. In this situation, some embodiments will enable perpendicular and/or tangential cut into the dura. In some embodiments, the perforating element can be made of a memory material (e.g., nitinol) which curves upon aperture egress, or the aperture can be coupled to an angled surface to provide an angle of attack to the dura greater than 1 degree (e.g., greater than 2 degrees, greater than 5 degrees, or greater than 10 degrees). In some embodiments, the pre-shaped element is sufficiently strong to deform the delivery catheter in an advantageous way to point towards the dura. This is possible given the lax nature of the epidural fat and compliant venous wall. In some embodiments, the pre-shaped element is flexible enough to cause minimal or no deformation to the delivery catheter, but upon emergence into the vascular lumen it acquires a curvature that is maintained while penetrating through the vascular wall and dura.

***Lateral Lumbar Vein Epidural Access.*** For the lumbar vein epidural approach, again, ascending lumbar vein on the left would be the most straightforward approach. Left common femoral vein access would be obtained. A 5Fr or 6Fr catheter would be advanced into the left ascending lumbar vein usually at an angle of 70-150 degrees (typically 90-120 degree) for access into the neural foramen. Once access to the foramen is achieved the micro-catheter (3-4Fr) is advanced over a micro-wire in the lateral epidural space between the neural foramina of two adjacent spinal level. Following this, the shapeable RF micro-wire is advanced into the micro-catheter and directed medially to penetrate the dura.

Given the lack of a step-off between the delivery micro-catheter and the wire, the catheter is easily advanced into the thecal sac and position is confirmed with a contrast injection. The perforating element can generate a slit larger than the element itself to facilitate advancement of the surrounding co-axial element through the vascular wall, the extravascular tissue and the dura.

***Ventral Lumbar Epidural Vein Access.*** For the ventral lumbar epidural vein approach Left common femoral vein access would be obtained. A 5Fr catheter would be advanced into the left ascending lumbar vein. The catheter could be angle tipped or contain a steerable component to allow it to access the lumbar vein at an angle of 70-150 degrees (typically 90-120 degree) which is the angle of approach from the ascending lumbar vein to the foraminal vein. Following this, the delivery micro-catheter is directed into the ventral epidural venous plexus which lays ANTERIOR to the thecal sac.

Once the microcatheter is in position, the steerable RF microwire is pointed posterior using either the lateral plane or a flat panel CT guidance. There are no critical neurological structures in this location as the neural foramina contain the nerve roots. Once the microwire perforates the dura the catheter is advanced into the thecal sac.

Thoracic vein Access. The abovementioned lumbar vein approaches allow for shunt catheter delivery into the lumbar spine and placement of a shunt catheter which will equilibrate with the left common iliac vein and the CSF will be draining into the pelvic veins of the left common iliac vein.

Another approach that can be attempted is a thoracic vein approach. Access to the thoracic intercostal veins is performed via the azygos vein. A 6Fr catheter can be placed in the paraspinal/intercostal vein. Following this, our shunt delivery micro-catheter is advanced into the foraminal vein and will then enter the epidural venous plexus and be tracked to the lumbar epidural venous plexus below the level of L2. Once the target level is reached, the penetration of the dural can be performed using either the Lateral Lumbar Vein Epidural Approach explained above or the Ventral Lumbar Epidural Vein approach.

Following positioning of the catheter and delivery of the shunt catheter, the shunt catheter will then drain into the azygous vein or jugular vein rather than the common iliac vein/ascending lumbar vein. This introduces a different hemodynamic paradigm which affects shunt design as the pressure in the azygous vein ranges for 1-10 mmHg (133-1333 Pa). whereas the common iliac vein ranges from 10-20 mmHg (1333-2666 Pa).

By comparison to known CSF diversion technologies, the catheter devices 10, 110, 210 and non-claimed methods 1100 described herein are safer procedures, easier to perform, and faster to complete, and provide more potential access sites. The percutaneous and endovascular CSF diversion technologies described herein introduce CSF diverting catheters through veins of the spine that get in close proximity to the subarachnoid space (e.g., less than 1 mm away in some embodiments) without risk of injury to the spinal cord or brain of the patient. The lower spinal canal is more capacious than the CP angle (used in known methods) and has less critical neurological structures at risk of injury during the procedure.

The thoracic spinal level offers numerous access sites at the nerve sleeve level that can be coupled with mechanisms to control the depth of perforation resulting in effective access to the CSF with low risk or neurogenic injury. The upper cervical region offers access to the large cisterna magna.

Additionally, accessing the spinal epidural space from a femoral, jugular or other peripheral vein puncture is a much easier procedure. Additionally, the pressure in the common iliac vein is usually in the physiological range of normal intracranial pressure (e.g., 1 to 20 mmHg, 133 to 2666 Pa) so equilibrium of CSF drainage may achieve normal intracranial pressure. In other words, fluidly connecting the CSF space with the venous system mimics the physiological process of CSF flow.

The catheter devices and non-claimed methods described herein permit navigation of the spinal venous system and permit a user to reach any vein in the spine in a matter of 20 to 30 minutes. The catheter 22 of the first example catheter device 10, for example, would be retrievable via endovascular means. Modifications could be made to the catheter in vivo, which allows for reduction or increase in CSF drainage. Alternatively, a CSF diverting stent could be introduced in the lower spinal CSF space that allows CSF to be drained into the epidural and paraspinal venous space.

## Claims

1. A shunt catheter device for draining cerebrospinal fluid (CSF) into a venous system of a lower spinal region, the shunt catheter device comprising:
a sheath (14) having an interior volume (18);
a catheter (22) disposed in the interior volume (18) of the sheath (14) and movable relative to the sheath (14);
a first flange (26) coupled to the catheter (22) and disposed between the catheter (22) and the sheath (14); and
a second flange (30) proximally located relative to the first flange (26) and disposed between the catheter (22) and the sheath (14),
wherein the first and second flanges are expandable between a compressed configuration when the first and second flanges (26, 30) are disposed in the interior volume (18) of the sheath (14), and an expanded configuration when the first and second flanges (26, 30) are externally disposed relative to the sheath (14), and
wherein the first flange (26) is separably deliverable relative to the second flange (30).

2. The shunt catheter device of claim 1, wherein the first and second flanges are a Nitinol braided material.

3. The shunt catheter device of claim 1 or 2, wherein the first and second flanges (26, 30) are attached to the catheter (22).

4. The shunt catheter device of any one of claims 1 through 3, further comprising a check valve (56) operably coupled to the catheter (22) and disposed downstream from the first and second flanges (26, 30).

5. The shunt catheter device of any one of claims 1 through 4, wherein the catheter (22) has a length in a range of 10 cm to 20 cm.

6. The shunt catheter device of any one of claims 1 through 5, wherein the catheter (22) has an inner diameter in a range of 0.5 mm to 2.0 mm.

7. The shunt catheter device of any one of claims 1 through 6, wherein one or more of the first and second flanges (26, 30) has an outer diameter in a range of 2.5 mm to 5 mm in the expanded configuration.

8. The shunt catheter device of any one of claims 1 through 7, wherein one or more of the first and second flanges (26, 30) is coupled to the catheter (22) by spot-welding.

9. The shunt catheter device of any one of claims 1 through 7, wherein one or more of the first and second flanges (26, 30) is coupled to the catheter (22) by stitching.

## Patentansprüche

1. Shunt-Kathetervorrichtung zum Ablassen von Liquor (CSF) in ein venöses System eines unteren Wirbelsäulenbereichs, wobei die Shunt-Kathetervorrichtung Folgendes umfasst:
eine Hülle (14) mit einem Innenvolumen (18);
einen Katheter (22), der im Innenvolumen (18) der Hülle (14) angeordnet und relativ zur Hülle (14) beweglich ist;
einen ersten Flansch (26), der mit dem Katheter (22) gekoppelt und zwischen dem Katheter (22) und der Hülle (14) angeordnet ist; und
einen zweiten Flansch (30), der proximal relativ zum ersten Flansch (26) angeordnet und zwischen dem Katheter (22) und der Hülle (14) angeordnet ist,
wobei der erste und zweite Flansch zwischen einer komprimierten Konfiguration, wenn der erste und zweite Flansch (26, 30) im Innenvolumen (18) der Hülle (14) angeordnet sind, und einer expandierten Konfiguration, wenn der erste und zweite Flansch (26, 30) relativ zur Hülle (14) außerhalb angeordnet sind, expandierbar sind und
wobei der erste Flansch (26) relativ zum zweiten Flansch (30) trennbar lieferbar ist.

2. Shunt-Kathetervorrichtung nach Anspruch 1, wobei der erste und zweite Flansch ein Nitinol-geflochtenes Material sind.

3. Shunt-Kathetervorrichtung nach Anspruch 1 oder 2, wobei der erste und zweite Flansch (26, 30) am Katheter (22) angebracht sind.

4. Shunt-Kathetervorrichtung nach einem der Ansprüche 1 bis 3, ferner umfassend ein Rückschlagventil (56), das mit dem Katheter (22) wirkgekoppelt und dem ersten und zweiten Flansch (26, 30) nachgelagert angeordnet ist.

5. Shunt-Kathetervorrichtung nach einem der Ansprüche 1 bis 4, wobei der Katheter (22) eine Länge im Bereich von 10 cm bis 20 cm aufweist.

6. Shunt-Kathetervorrichtung nach einem der Ansprüche 1 bis 5, wobei der Katheter (22) einen Innendurchmesser im Bereich von 0,5 mm bis 2,0 mm aufweist.

7. Shunt-Kathetervorrichtung nach einem der Ansprüche 1 bis 6, wobei der erste und/oder zweite Flansch (26, 30) in der expandierten Konfiguration einen Außendurchmesser im Bereich von 2,5 mm bis 5 mm aufweist/aufweisen.

8. Shunt-Kathetervorrichtung nach einem der Ansprüche 1 bis 7, wobei der erste und/oder zweite Flansch (26, 30) durch Punktschweißen mit dem Katheter (22) gekoppelt ist/sind.

9. Shunt-Kathetervorrichtung nach einem der Ansprüche 1 bis 7, wobei der erste und/oder zweite Flansch (26, 30) durch Nähen mit dem Katheter (22) gekoppelt ist/sind.

## Revendications

1. Dispositif de cathéter de dérivation pour drainer du liquide céphalorachidien (CSF) dans un système veineux d'une région vertébrale inférieure, le dispositif de cathéter de dérivation comprenant :
une gaine (14) ayant un volume intérieur (18) ;
un cathéter (22) disposé dans le volume intérieur (18) de la gaine (14) et mobile par rapport à la gaine (14) ;
une première bride (26) couplée au cathéter (22) et disposée entre le cathéter (22) et la gaine (14) ; et
une seconde bride (30) située proximalement par rapport à la première bride (26) et disposée entre le cathéter (22) et la gaine (14),
les première et seconde brides étant déployables entre une configuration comprimée lorsque les première et seconde brides (26, 30) sont disposées dans le volume intérieur (18) de la gaine (14), et une configuration déployée lorsque les première et seconde brides (26, 30) sont disposées extérieurement par rapport à la gaine (14), et
la première bride (26) pouvant être délivrée de manière séparable par rapport à la seconde bride (30).

2. Dispositif de cathéter de dérivation selon la revendication 1, les première et seconde brides étant un matériau tressé de Nitinol.

3. Dispositif de cathéter de dérivation selon la revendication 1 ou 2, les première et seconde brides (26, 30) étant fixées au cathéter (22).

4. Dispositif de cathéter de dérivation selon l'une quelconque des revendications 1 à 3, comprenant en outre un clapet anti-retour (56) couplé de manière opérationnelle au cathéter (22) et disposé en aval des première et seconde brides (26, 30).

5. Dispositif de cathéter de dérivation selon l'une quelconque des revendications 1 à 4, le cathéter (22) ayant une longueur dans une plage de 10 cm à 20 cm.

6. Dispositif de cathéter de dérivation selon l'une quelconque des revendications 1 à 5, le cathéter (22) ayant un diamètre intérieur dans une plage de 0,5 mm à 2,0 mm.

7. Dispositif de cathéter de dérivation selon l'une quelconque des revendications 1 à 6, une ou plusieurs des première et seconde brides (26, 30) ayant un diamètre extérieur dans une plage de 2,5 mm à 5 mm dans la configuration déployée.

8. Dispositif de cathéter de dérivation selon l'une quelconque des revendications 1 à 7, une ou plusieurs des première et seconde brides (26, 30) étant couplées au cathéter (22) par soudage par points.

9. Dispositif de cathéter de dérivation selon l'une quelconque des revendications 1 à 7, une ou plusieurs des première et seconde brides (26, 30) étant couplées au cathéter (22) par couture.
